# EUROPÄISCHE PATENTSCHRIFT

(11) **EP 3 799 565 B1**
(45) Veröffentlichungstag und Bekanntmachung des Hinweises auf die Patenterteilung: **04.01.2023**
(21) Anmeldenummer: 19746444.9
(22) Anmeldetag: 12.07.2019
(51) Int. Cl.: A61F 11/00, A61M 3/02, A61F 7/12

(54) **AUFSATZ FÜR EINE VORRICHTUNG ZUR GENERIERUNG EINES LUFTSTROMES IN DEN ÄUSSEREN GEHÖRGANG**
SPRAY HEAD FOR A DEVICE FOR PRODUCING A STREAM OF AIR IN THE OUTER EAR
EMBOUT POUR UN DISPOSITIF DE GÉNÉRATION D'UN FLUX D'AIR DANS LE CONDUIT AUDITIF EXTERNE

(30) Priorität: 13.07.2018 EP 18183461
(43) Veröffentlichungstag der Anmeldung: 07.04.2021
(73) Patentinhaber: EARBREEZE GMBH, 1010 Wien (AT)
(72) Erfinder: KURSCHEL, Martin, 1010 Wien (AT)
(74) Vertreter: KLIMENT & HENHAPEL
(86) Internationale Anmeldenummer: PCT/EP2019/068922
(87) Internationale Veröffentlichungsnummer: WO 2020/012032

(56) Entgegenhaltungen:
- EP-A1- 0 243 261
- WO-A1-2012/166801
- DE-T2- 69 214 690
- US-A1- 2011 099 832
- US-A1- 2015 090 191
- US-A1- 2018 125 345

## Beschreibung

### GEBIET DER ERFINDUNG

Die vorliegende Erfindung betrifft einen Aufsatz für eine Vorrichtung zur Generierung eines Luftstromes, vorzugsweise eines Warmluftstromes, oder zur Abgabe eines Fluids, welcher Aufsatz dazu eingerichtet ist, zumindest abschnittsweise in einen äußeren Gehörgang eines menschlichen oder tierischen Ohres eingeführt zu werden, um Feuchtigkeitsansammlungen in dem äußeren Gehörgang zu trocknen.

Des Weiteren betrifft die Erfindung ein Ohrentrocknungsgerät umfassend eine Vorrichtung zur Generierung eines Luftstromes sowie den erfindungsgemäßen Aufsatz.

Darüber hinaus betrifft die Erfindung ein Gerät zur pflegenden oder therapeutischen Behandlung des äußeren Gehörgangs eines menschlichen oder tierischen Ohres.

### STAND DER TECHNIK

Patienten, die unter chronisch feuchten Gehörgängen leiden - sei es auf Grund einer Radikalhöhle, einer Trommelfellperforation oder einer anderen Erkrankung des äußeren Gehörgangs - oder auch Träger von Hörgeräten sind häufig mit dem Problem der effizienten Entfernung von Feuchtigkeit aus dem äußeren Gehörgang des Ohres konfrontiert. Zudem kann Wasser in der Badewanne, unter der Dusche oder beim Schwimmen in den Gehörgang gelangen, was zu einem verminderten Hörvermögen führen kann. Auch können Bakterien, Keime und Schmutz so leichter in den äußeren Gehörgang gelangen und dort Entzündungen hervorrufen, was eine rasche Entfernung dieser Flüssigkeitsansammlungen erforderlich macht.

Aus dem Stand der Technik sind jedoch keine Vorrichtungen bekannt, mit Hilfe derer eine solche Entfernung auf gefahrlose und effiziente Weise möglich ist. Beispielsweise ist es bekannt, einen Luftstrom in den Gehörgang einzuleiten, durch den der äußere Gehörgang getrocknet werden soll. Ein in den äußeren Gehörgang einführbarer Aufsatz für eine Vorrichtung zur Generierung eines Luftstromes ist aus EP 0937422 B1 bekannt. Zum einen führt der darin vorgeschlagene Aufsatz die in den äußeren Gehörgang eingeleitete Luft aber direkt auf das Trommelfell des behandelten Ohres, wodurch es zu Verletzungen und bleibenden Schäden kommen kann; zum anderen kann der Aufsatz aufgrund seiner Bauform nicht oder nur wenige Millimeter tief in den Gehörgang des Patienten eingeschoben werden, wodurch insbesondere Feuchtigkeitsansammlungen, die sich im trommelfellseitigen Endbereich des üblicherweise 2 bis 2,5 cm langen Gehörganges befinden, nicht oder nur nach längerer Anwendungsdauer und/oder höherer Intensität des Luftstromes entfernt werden können.

In analoger Weise kann es bei der Befreiung des äußeren Gehörgangs von Cerumen mittels eines Fluids zu Verletzungen des Trommelfells kommen, wenn das Fluid direkt auf das Trommelfell geleitet wird. Zugleich können Cerumen-Rückstände im äußeren Gehörgang verbleiben und die Reinigung des äußeren Gehörgangs somit nur unzufrieden stellend betrieben werden, wenn das Fluid nicht den gesamten äußeren Gehörgang erreichen kann. Eine Vorrichtung zur Reinigung des äußeren Gehörgangs ist beispielsweise aus EP 0 243 261 A1 bekannt.

Auch im Rahmen der Abgabe eines Fluids in den äußeren Gehörgang zum Zwecke der Pflege bzw. therapeutischen Behandlung des äußeren Gehörgangs kann es zu Verletzungen des Trommelfells kommen, wenn das Fluid direkt auf das Trommelfell geleitet wird. Der pflegende bzw. therapeutische Effekt einer solchen Behandlung des äußeren Gehörgangs mittels eines Fluids kann zudem nicht sein Maximum erreichen, wenn das Fluid nicht den gesamten äußeren Gehörgang erreichen kann.

### AUFGABE DER ERFINDUNG

Daher ist es eine Aufgabe der vorliegenden Erfindung, einen Aufsatz für eine Vorrichtung zur Generierung eines Luftstromes, vorzugsweise eines Warmluftstromes, bereitzustellen, welcher Aufsatz eine rasche und effiziente Entfernung von Feuchtigkeit aus dem äußeren Gehörgang eines Menschen oder eines Tieres mittels eines Luftstromes ermöglicht, ohne dabei das Trommelfell der Gefahr einer Beschädigung auszusetzen.

Außerdem soll der bereitgestellte Aufsatz auf unkomplizierte Weise an verschiedene Gehörgänge anpassbar sein, einen einfachen Aufbau aufweisen und in der Herstellung kostengünstig sein.

Die Effizienz von Ohrentrocknungsgeräten, welche den erfindungsgemäßen Aufsatz umfassen, soll zudem deutlich erhöht werden. Das erfindungsgemäße Ohrentrocknungsgerät soll ein merkbares Trocknungsergebnis bereits nach einer Anwendungsdauer von 15 Sekunden liefern. Eine vollständige Trocknung soll durch eine Anwendungsdauer von etwa 30 Sekunden oder weniger erreicht werden können.

Zudem ist es eine Aufgabe der vorliegenden Erfindung, einen Aufsatz für eine Vorrichtung zur Abgabe eines Fluids bereitzustellen, welcher Aufsatz eine rasche und effiziente Entfernung von Cerumen aus dem äußeren Gehörgang eines Menschen oder eines Tieres mittels eines Fluids ermöglicht, ohne dabei das Trommelfell der Gefahr einer Beschädigung auszusetzen.

Schließlich ist es eine Aufgabe der vorliegenden Erfindung, einen Aufsatz für eine Vorrichtung zur Abgabe eines Fluids bereitzustellen, welcher Aufsatz eine rasche und effiziente pflegende oder therapeutische Behandlung des äußeren Gehörgangs eines Menschen oder eines Tieres mittels eines Fluids ermöglicht, ohne dabei das Trommelfell der Gefahr einer Beschädigung auszusetzen.

### DARSTELLUNG DER ERFINDUNG

Eine der Erfindung zugrunde liegende Aufgabe wird erfindungsgemäß durch einen Aufsatz für eine Vorrichtung zur Erzeugung eines Luftstromes, vorzugsweise eines Warmluftstromes, oder zur Abgabe eines Fluids gelöst, welcher Aufsatz dazu eingerichtet ist, zumindest abschnittsweise in einen äußeren Gehörgang eines menschlichen oder tierischen Ohres eingeführt zu werden, wobei der Aufsatz einen Verbindungsstutzen zur Anbindung an die Vorrichtung, sowie eine von dem Verbindungsstutzen abstehende, zungenartig ausgebildete Leitschaufel mit einem Leitschaufelboden zur Führung von aus der Vorrichtung ausströmender Luft oder von der Vorrichtung abgegebenem Fluid aufweist, und wobei die Leitschaufel dem Verbindungsstutzen in Strömungsrichtung des Luftstromes oder Fluids nachgeordnet ist, wobei die zungenartig ausgebildete Leitschaufel zwei von dem Leitschaufelboden abstehende Leitschaufelwangen, nämlich eine erste Leitschaufelwange und eine zweite Leitschaufelwange, aufweist, wobei Leitschaufelboden und Leitschaufelwangen einen Leitkanal oder einen Teil eines Leitkanals für die aus der Vorrichtung ausströmende Luft ausbilden, und wobei die erste Leitschaufelwange und die zweite Leitschaufelwange, insbesondere einander gegenüberliegende Abschnitte der Leitschaufelwangen, jeweils eine unterschiedliche Höhe aufweisen. Der Verbindungsstutzen kann dabei unterschiedlich ausgestaltet sein. Er kann beispielsweise ein wesentliches Element des Aufsatzes ausbilden und diesem Stabilität verleihen. Er kann aber auch lediglich einen Abschnitt des Aufsatzes ausmachen, der die Anbindung des Aufsatzes an die Vorrichtung ermöglicht ohne dabei wesentlich zur Stabilität des Aufsatzes beizutragen.

Erfindungsgemäß wird der Luft- oder Fluidstrom, welchen die Vorrichtung bereitstellt (im Folgenden wird der Einfachheit nur der Luftstrom genannt), über den erfindungsgemäßen Aufsatz in den äußeren Gehörgang eingeleitet und dabei durch die Leitschaufel so gelenkt, dass sich innerhalb des äußeren Gehörgangs eine für die erwünschte Trocknung besonders vorteilhafte Strömung einstellt. Der Luftstrom strömt dabei über den Verbindungsstutzen in den Aufsatz ein, durchströmt zunächst den Verbindungsstutzen und wird in weiterer Folge über den Leitschaufelboden der Leitschaufel in Richtung des äußeren Gehörgangs geführt. Durch die erfindungsgemäß vorgesehenen Leitschaufelwangen kann eine gezielte und kontrollierte Führung des Luftstromes entlang der Leitschaufel sowie eine kontrollierte Abgabe des Luftstromes an den äußeren Gehörgang sichergestellt werden. Zudem wird der Luftstrom durch den erfindungsgemäßen Aufsatz so gelenkt, dass sich im Inneren des äußeren Gehörgangs näherungsweise ein hinsichtlich der Trocknung besonders günstiges Wirbelfeld ausbildet. Dieses Wirbelfeld kann sich - je nachdem, wie weit der Aufsatz in den äußeren Gehörgang des Anwenders eingeführt wird - entweder aufgrund eines Dralls ergeben, welchen der Luftstrom aufgrund der Leitschaufel erfährt, und/oder aufgrund von Reflexion des Luftstromes an zum Trommelfell führenden Seitenwänden des äußeren Gehörgangs ausbilden. Der Luftstrom kann dadurch nicht direkt auf das Trommelfell auftreffen, sondern nur auf die Seitenwände des äußeren Gehörgangs und an diesen Seitenwänden, vorzugsweise mehrmals, reflektiert werden, bevor er entlang des Trommelfells geleitet wird, so dass er an dessen Oberfläche entlang streicht. Beispielsweise kann der Luftstrom an einer oberen Seitenwand des Gehörgangs auftreffen, dort reflektiert werden und entlang des Trommelfells in Richtung einer unteren Seitenwand des äußeren Gehörgangs geleitet werden, wo der Luftstrom ein weiteres Mal reflektiert und schließlich in Richtung der Ohrmuschel gefördert wird. Es ist auch möglich, dass der Luftstrom mehrfach an den Seitenwänden des äußeren Gehörgangs reflektiert wird, bevor der Luftstrom das Trommelfell erreicht. Insbesondere können mittels des erfindungsgemäßen Aufsatzes Feuchtigkeitsansammlungen auch aus dem Recessus meatus acustici externi entfernt werden - also jener Mulde, welche sich unmittelbar vor dem Trommelfell zwischen dem schräg nach unten auf das Trommelfell zulaufenden Boden des äußeren Gehörgangs und dem diesen Boden überdachenden, schrägstehenden Trommelfell ergibt. Die Verwirbelung des Luftstromes, sei sie nun durch die Drallgebung und/oder durch Reflexion an den zum Trommelfell führenden Seitenwänden des äußeren Gehörgangs, führt dazu, dass der Luftstrom über das Trommelfell und den Recessus meatus acustici externi streicht, nicht aber frontal auf das Trommelfell auftrifft. Aufgrund des durch den erfindungsgemäßen Aufsatz bedingten Strömungsverlaufes des Luftstromes im Inneren des äußeren Gehörgangs kommt es zu besonders kurzen Anwendungszeiten, die für eine zufriedenstellende Trocknung erforderlich sind. Beispielsweise kann ein merkbarer Trocknungseffekt bereits nach 15 Sekunden verzeichnet werden, wenn ein erfindungsgemäßes Ohrtrocknungsgerät - wie es weiter unten beschrieben ist - mit dem erfindungsgemäßen Aufsatz zur Anwendung kommt. Um den äußeren Gehörgang vollständig zu trocknen - also den Feuchtegehalt im Gehörgang wieder auf das übliche Maß zu reduzieren, ist eine Anwendungsdauer von etwa 30 Sekunden oder weniger ausreichend.

Außerdem hat sich gezeigt, dass der erfindungsgemäße Aufsatz auch eine besonders schonende und zugleich effektive Entfernung von Cerumen aus dem äußeren Gehörgang ermöglicht, wenn der Aufsatz in Verbindung mit einer Vorrichtung zur Abgabe eines Fluids verwendet wird. Auf Grund der erfindungsgemäßen Leitschaufel bildet das Fluid, beispielsweise Wasser, im Inneren des äußeren Gehörgangs einen für die Reinigung besonders günstigen Strömungsverlauf aus. Analog zu dem oben beschriebenen Luftstrom wird auch das Fluid in ein näherungsweises Wirbelfeld gezwungen, wenn es in den äußeren Gehörgang einströmt, sodass einerseits ein direktes Auftreffen des Fluids auf das Trommelfell unterbunden wird und gleichzeitig durch die Verwirbelung und/oder Reflexion des Fluids an den Seitenwänden des äußeren Gehörgangs eine besonders effektive Entfernung von Cerumen aus dem äußeren Gehörgang, insbesondere von an den Seitenwänden ansitzendem Cerumen, sichergestellt ist.

Weiters hat sich herausgestellt, dass der erfindungsgemäße Aufsatz auch eine besonders schonende und zugleich effektive pflegende oder therapeutische Behandlung des äußeren Gehörgangs ermöglicht, wenn der Aufsatz in Verbindung mit einer Vorrichtung zur Abgabe eines Fluids verwendet wird. Auf Grund der erfindungsgemäßen Leitschaufel bildet das Fluid im Inneren des äußeren Gehörgangs einen für die pflegende oder therapeutische Behandlung des äußeren Gehörgangs besonders günstigen Strömungsverlauf aus. Dabei können Verletzungen des Trommelfells vermieden und eine Maximierung des pflegenden oder therapeutischen Effektes erzielt werden, da das zur pflegenden oder therapeutischen Behandlung verwendete Fluid nicht direkt auf das Trommelfell gelenkt wird und auf Grund der erfindungsgemäßen Verwirbelung und/oder Reflexion des Fluids die Seitenwände des äußeren Gehörgangs besonders gut erreichen kann.

Gemäß einer bevorzugten Ausführungsform ist es vorgesehen, dass die erste Leitschaufelwange von einem ersten längsseitigen Rand des Leitschaufelbodens absteht und die zweite Leitschaufelwange von einem dem ersten längsseitigen Rand gegenüberliegenden zweiten längsseitigen Rand des Leitschaufelbodens absteht. Alternativ kann es auch vorgesehen sein, dass die erste Leitschaufelwange durch den ersten längsseitigen Rand des Leitschaufelbodens und die zweite Leitschaufelwange durch den zweiten längsseitigen Rand des Leitschaufelbodens ausgebildet ist.

Dabei begrenzen die Leitschaufelwangen den Leitschaufelboden an dessen einander gegenüberliegenden Längsseiten und verhindern weitestgehend, dass der zur Trocknung dienende Luftstrom aus dem Leitkanal in andere Bereiche des Aufsatzes gelangen kann, bevor er zur bestimmungsgemäßen Trocknung in den äußeren Gehörgang gelangt. So können beispielsweise Leitkanal und ein Abluftkanal des Aufsatzes (siehe weiter unten) durch die Leitschaufel fluidisch voneinander isoliert bzw. getrennt werden. Außerdem können die Leitschaufelwangen die Ausbildung des Wirbelfeldes im äußeren Gehörgang unterstützen. Besonders vorteilhafte Trocknungseffekte lassen sich erzielen, wenn die Leitschaufelwangen über die gesamte Länge der Leitschaufel verlaufen. Die Leitschaufelwangen können einstückig mit dem Leitschaufelboden gefertigt sein. Die Leitschaufelwangen können ein Querschnittsprofil mit im Vergleich zum zwischen den Leitschaufelwangen liegenden Leitschaufelboden unterschiedlicher Krümmung aufweisen. Der Übergang zwischen Leitschaufelboden und Leitschaufelwangen kann fließend sein und sich etwa lediglich durch eine Veränderung in der Steigung bzw. Krümmung des Querschnittsprofils des Leitschaufelbodens äußern. Dies kann eine eindeutige Unterscheidung zwischen Leitschaufelboden und Leitschaufelwangen erschweren. Beispielsweise können Leitschaufelboden und Leitschaufelwangen derart angeordnet sein, dass ein durch Leitschaufelboden und Leitschaufelwangen ausgebildeter Gesamtquerschnitt der Leitschaufel im Wesentlichen U-förmig ausgebildet ist.

Während es zur Verwirklichung des oben beschriebenen, erfindungsgemäßen Effekts, der eine schonende Trocknung des Trommelfells ermöglicht, grundsätzlich ausreichend ist, die zungenartig ausgebildete Leitschaufel in den äußeren Gehörgang einzuführen und entweder die durch die Leitschaufel bewirkte Verwirbelung des Luftstroms oder die ebenfalls durch die Leitschaufel bewirkte Reflexion und/oder Verwirbelung an den Wänden des äußeren Gehörgangs ausnützt, kann es vorteilhaft sein, zusätzlich einen Ohrtrichter für den Aufsatz vorzusehen.

Gemäß einer bevorzugten Ausführungsform des erfindungsgemäßen Aufsatzes ist es daher vorgesehen, dass der Aufsatz einen Ohrtrichter umfasst, welcher Ohrtrichter die zungenartig ausgebildete Leitschaufel zumindest abschnittsweise, vorzugsweise gänzlich, der Länge nach umhüllt und eine proximale Öffnung aufweist, mit welcher proximalen Öffnung der Aufsatz bei bestimmungsgemäßer Verwendung in den äußeren Gehörgang eingeführt oder angesetzt ist, sodass die proximale Öffnung die dem Trommelfell nächstgelegene Öffnung des Ohrtrichters darstellt, wobei der Ohrtrichter an den Verbindungsstutzen anschließt und von diesem absteht, oder der Verbindungsstutzen einen Endabschnitt des Ohrtrichters ausbildet. Bei bestimmungsgemäßer Verwendung des Aufsatzes zur Trocknung des äußeren Gehörgangs ist der Aufsatz mit der proximalen Öffnung des Ohrtrichters in den äußeren Gehörgang des Anwenders eingeführt oder an diesen angesetzt. Der Begriff *proximal* bezieht sich dabei und im Folgenden auf die Lage eines bestimmten Elementes relativ zu dem Trommelfell des Anwenders. Konkret ist mit der proximalen Öffnung die dem Trommelfell des Anwenders nächstgelegene Öffnung des Ohrtrichters gemeint.

Der Ohrtrichter dient dabei als jener Teil des Aufsatzes, welcher in direkten Kontakt mit dem äußeren Gehörgang bzw. den Seitenwänden desselben gebracht wird, wenn der Aufsatz bestimmungsgemäß verwendet und in den äußeren Gehörgang des Anwenders eingeführt wird. Der Ohrtrichter kann an den Verbindungsstutzen des Aufsatzes anschließen und von diesem abstehen bzw. kann der Verbindungsstutzen einen Endabschnitt des Ohrtrichters ausbilden. Die Leitschaufel ist zumindest abschnittsweise innerhalb des, vorzugsweise hülsenförmigen, Ohrtrichters angeordnet. Vorzugsweise ragt die Leitschaufel nicht aus dem Ohrtrichter heraus. Der Ohrtrichter ermöglicht einerseits eine leichte Aufweitung und eine für die Trocknung vorteilhafte Positionierung - nämlich eine leichte Begradigung - des üblicherweise schräg verlaufenden äußeren Gehörgangs, wenn der Aufsatz mit dem Ohrtrichter in den äußeren Gehörgang eines Menschen oder eines Tieres eingeführt ist; andererseits erlaubt der Ohrtrichter aber auch eine einfache Anpassung des Aufsatzes an verschiedene Gegebenheiten, wie die Anwendung bei Erwachsenen oder Kindern - etwa durch entsprechende Wahl von Form und Größe des Ohrtrichters. Die Länge des Ohrtrichters kann auf den Längsverlauf der Leitschaufel abgestellt sein, um die Ausbildung der oben beschriebenen Verwirbelungen und Reflexionen im äußeren Gehörgang zu unterstützen. Bei Ausführungsformen des Aufsatzes mit Ohrtrichter können etwaige ungewollte Reflexionen des Luftstromes an einer Innenwand des Ohrtrichters durch die Leitschaufelwangen vermieden werden. Dabei können die Leitschaufelwangen den Ohrtrichter beispielsweise über die gesamte Länge des Leitschaufelbodens oder auch nur in einem oder mehreren Längsabschnitten des Leitschaufelbodens kontaktieren. Alternativ können die Leitschaufelwangen aber auch von dem Ohrtrichter beabstandet sein. Wenn die Leitschaufelwangen über ihre gesamte Längserstreckung den Ohrtrichter kontaktieren, kann ein solches Überströmen von Luft aus dem Leitkanal in andere Bereiche, etwa einen Abluftkanal des Aufsatzes (siehe weiter unten), vollständig verhindert werden.

Gemäß einer bevorzugten Ausführungsform des erfindungsgemäßen Aufsatzes ist es vorgesehen, dass der Leitschaufelboden eines ersten Abschnitts der Leitschaufel einen Längsquerschnittsverlauf aufweist, der in eine erste Richtung gekrümmt ist, und der Leitschaufelboden eines zweiten Abschnitts der Leitschaufel einen Längsquerschnittsverlauf aufweist, der in eine zweite Richtung gekrümmt ist, und die beiden gekrümmten Abschnitte zu einander entgegengesetzt gekrümmt verlaufen.

Durch diesen speziellen Verlauf der Leitschaufel bzw. des Leitschaufelbodens wird eine besonders günstige Strömungsverteilung des Luftstromes innerhalb des äußeren Gehörganges erzielt. Bei dieser Ausführungsform der Erfindung konnten besonders gute Ergebnisse bei der Trocknung erzielt werden, insbesondere im Vergleich mit Aufsätzen mit geradem Längsquerschnittsverlauf. Besonders vorteilhaft ist es, wenn - von dem Verbindungsstutzen aus betrachtet - die Steigung einer an den Leitschaufelboden angelegten Tangente im ersten Abschnitt des Leitschaufelbodens mit dem Abstand von dem Verbindungsstutzen abnimmt und im zweiten Abschnitt des Leitschaufelbodens mit dem Abstand von dem Verbindungsstutzen zunimmt.

Gemäß einer weiteren bevorzugten Ausführungsform der Erfindung ist es vorgesehen, dass der erste Abschnitt und der zweite Abschnitt unmittelbar ineinander übergehen. Insbesondere ist es strömungstechnisch günstig, wenn der erste Abschnitt und der zweite Abschnitt des Leitschaufelbodens nicht durch eine Unstetigkeit voneinander getrennt sind sondern glatt ineinander übergehen.

Dies führt zu einem für die erwünschten Trocknungseffekte besonders günstigen Strömungsverlauf des zur Trocknung dienenden Luftstromes innerhalb des äußeren Gehörgangs.

Gemäß einer weiteren bevorzugten Ausführungsform der Erfindung ist es vorgesehen, dass, bei Betrachtung eines Längsquerschnitts des Aufsatzes, der Leitschaufelboden eine Längsachse des Aufsatzes schneidet, welche Längsachse durch den Mittelpunkt eines auf den Längsquerschnitt normal stehenden Querschnittes des Verbindungsstutzens verläuft.

Die zungenartig ausgebildete Leitschaufel des Aufsatzes schneidet demnach die durch den Mittelpunkt des Querschnittes des Verbindungsstutzens verlaufende Längsachse. Erfindungsgemäß setzt dies voraus, dass die Leitschaufel in einer Seitenansicht des Aufsatzes zumindest abschnittsweise schräg zu dieser Längsachse verläuft. Vorzugsweise kann die Leitschaufel über ihre Länge in Strömungsrichtung gesehen eine nicht verschwindende Steigung aufweisen. Dadurch, dass die Leitschaufel, insbesondere der Längsquerschnitt des Leitschaufelbodens, die Längsachse des Aufsatzes quer schneidet, wird der Luftstrom so gelenkt, dass er nicht direkt auf das Trommelfell des Anwenders auftrifft.

Gemäß einer weiteren bevorzugten Ausführungsform des erfindungsgemäßen Aufsatzes ist es vorgesehen, dass, bei Betrachtung eines Längsquerschnitts des Aufsatzes, der Leitschaufelboden auch eine Längsachse des Ohrtrichters schneidet, welche Längsachse durch den Mittelpunkt einer proximalen Öffnung des Ohrtrichters verläuft. Sofern der Ohrtrichter rotationssymmetrisch ist - was abgesehen von einer Auslassöffnung im Mantel des Ohrtrichters vorzugsweise der Fall ist - fällt die Längsachse des Ohrtrichters mit dessen Rotationsachse zusammen. Die Leitschaufel kann derart innerhalb des Ohrtrichters angeordnet sein, dass in einem Längsschnitt betrachtet, der Leitschaufelboden, die Längsachse des Ohrtrichters schneidet, wobei es sich als besonders effektiv für die zu erzielenden Verwirbelungen bzw. Reflexionen und damit für die zu erzielende Trocknung herausgestellt hat, wenn der Leitschaufelboden die Längsachse des Ohrtrichters in seinem proximalen Endbereich schneidet.

Vorzugsweise fällt die Längsachse des Ohrtrichters mit der Längsachse des Aufsatzes zusammen. Dadurch kann der Aufbau des erfindungsgemäßen Aufsatzes besonders einfach gehalten werden.

Gemäß einer weiteren bevorzugten Ausführungsform des erfindungsgemäßen Aufsatzes ist es vorgesehen, dass die zungenartig ausgebildete Leitschaufel einen im Verbindungsstutzen verlaufenden Abschnitt aufweist.

Dies ist insbesondere dann von Vorteil, wenn der Aufsatz keinen oder einen abnehmbaren Ohrtrichter aufweist. Der Verbindungsstutzen dient in diesem Fall einerseits zur Anbindung an die Vorrichtung zur Erzeugung des Luftstroms, verleiht andererseits aber auch dem Aufsatz die erforderliche Stabilität, um die Leitschaufel zu tragen.

Um Verletzungen des Trommelfells und/oder der Seitenwand des äußeren Gehörgangs zu vermeiden, ist es bei einer weiteren bevorzugten Ausführungsform des erfindungsgemäßen Aufsatzes vorgesehen, dass die zungenartig ausgebildete Leitschaufel vollständig innerhalb des Ohrtrichters und/oder des Verbindungsstutzens angeordnet ist.

Bei Aufsätzen gemäß dieser Ausführungsform der Erfindung kann die Gefahr von Verletzungen des Trommelfells und/oder der Seitenwände des äußeren Gehörgangs des Anwenders minimiert werden, da die gesamte Leitschaufel im Inneren des Aufsatzes verborgen ist. Insbesondere können erster und zweiter Abschnitt der Leitschaufel innerhalb des Ohrtrichters und der im Verbindungsstutzen verlaufende Abschnitt der Leitschaufel innerhalb des Verbindungsstutzens angeordnet sein.

Gemäß einer weiteren bevorzugten Ausführungsform des Aufsatzes gemäß dieser Erfindung ist es vorgesehen, dass sich der Ohrtrichter von dem Verbindungsstutzen hin zu einer proximalen Öffnung des Ohrtrichters verjüngt.

Die proximale Öffnung hat vorzugsweise einen kreisrunden Umfang und einen Durchmesser von nicht mehr als 7 mm. Da somit die Form des Ohrtrichters zumindest in einem proximalen Abschnitt des Ohrtrichters, welcher in den Gehörgang eingeführt wird, an die Form des äußeren Gehörgangs angepasst ist, ermöglicht dies ein besonders einfaches und schmerzfreies Einführen des Aufsatzes in den äußeren Gehörgang. Der erfindungsgemäße Aufsatz kann somit bis auf etwa 15 mm, vorzugsweise 10 mm, weit an das Trommelfell des Anwenders herangeführt werden, wodurch die Ausbildung des weiter oben beschriebenen näherungsweisen Wirbelfeldes im äußeren Gehörgang unterstützt und eine Trocknung von Flüssigkeitsansammlungen im Recessus meatus acustici externi begünstigt werden kann. Die erzielbare minimale Entfernung vom Trommelfell kann je nach spezieller Eignung des Aufsatzes für die Verwendung an Männern, Frauen oder Kindern variieren.

Besonders bevorzugt ist ein proximales, dem Trommelfell nächstgelegenes Ende der Leitschaufel, vorzugsweise ein proximales Ende des Leitschaufelbodens, im Inneren der proximalen Öffnung des Ohrtrichters angeordnet.

Bei solchen Ausführungsformen der Erfindung schließt die Leitschaufel im Wesentlichen bündig mit dem Ohrtrichter ab. Dadurch kann der Luftstrom beim Austritt aus dem Aufsatz über die proximale Öffnung des Ohrtrichters in den äußeren Gehörgang besonders gut gelenkt werden. Sowohl ein Austrittswinkel, unter welchem der Luftstrom - bezogen auf die Längsachse des Ohrtrichters oder des Aufsatzes - aus dem Aufsatz ausströmt, als auch ein Drall, welcher dem Luftstrom bei Austritt aus dem Aufsatz mitgegeben werden soll, lassen sich bei dieser Ausführungsform besonders gut einstellen. Insbesondere kann so ein Einfallswinkel, unter welchem der Luftstrom auf die Seitenwände des äußeren Gehörgangs auftrifft, noch besser vorbestimmt werden.

Gemäß einer weiteren bevorzugten Ausführungsform des erfindungsgemäßen Aufsatzes ist es vorgesehen, dass das proximale Ende der Leitschaufel, vorzugsweise ein proximales Ende des Leitschaufelbodens, die proximale Öffnung des Ohrtrichters in eine Ausströmöffnung für aus dem Aufsatz in den Gehörgang ausströmende Luft sowie eine Einströmöffnung für aus dem Gehörgang in den Aufsatz einströmende Luft unterteilt.

Über die proximale Öffnung des Ohrtrichters strömt also einerseits der zur Trocknung dienende, trockene Luftstrom, vorzugsweise Warmluftstrom, aus dem Aufsatz in den äußeren Gehörgang ein - nämlich im Bereich der Ausströmöffnung; andererseits kann feuchte Luft aus dem äußeren Gehörgang über die proximale Öffnung aber auch wieder in den Aufsatz einströmen - nämlich im Bereich der Einströmöffnung. Somit ermöglicht der Ohrtrichter auch das Ausströmen der zur Trocknung verwendeten Luft aus dem Gehörgang und unterbindet die Entstehung eines Strömungsrückstaus innerhalb des Gehörgangs. Feuchte Luft kann den äußeren Gehörgang somit unmittelbar nach der Aufnahme von Feuchtigkeit und isoliert von den Seitenwänden des äußeren Gehörgangs wieder verlassen, was zu einem verbesserten Trocknungseffekt führt. Somit kann sichergestellt werden, dass der zur Trocknung verwendete Luftstrom im Bereich der proximalen Öffnung nicht mit feuchter Luft vermischt wird, bevor er in den äußeren Gehörgang eingeleitet wird. Das proximale Ende der Leitschaufel bzw. das proximale Ende des Leitschaufelbodens und/oder die Leitschaufelwangen kontaktieren in diesem Fall eine Innenseite des Ohrtrichters, um die Einströmöffnung und die Ausströmöffnung fluidisch voneinander zu trennen.

Gemäß einer weiteren bevorzugten Ausführungsform der Erfindung ist es vorgesehen, dass die zungenartig ausgebildete Leitschaufel ein Innenvolumen des Ohrtrichters in einen Zuluftkanal für aus dem Aufsatz in den Gehörgang strömende Luft sowie einen Abluftkanal für aus dem Gehörgang in den Aufsatz strömende Luft unterteilt, wobei vorzugsweise der Zuluftkanal den Verbindungsstutzen mit der Ausströmöffnung verbindet und wobei vorzugsweise der Abluftkanal die Einströmöffnung mit einem Auslass des Aufsatzes verbindet.

Hierdurch können der trockene Luftstrom und die feuchte aus dem äußeren Gehörgang austretende Luft im Aufsatz weitestgehend separat voneinander geführt werden. Der trockene Luftstrom, vorzugsweise Warmluftstrom, wird dabei zunächst durch die Vorrichtung erzeugt, durch den Verbindungsstutzen und über die Leitschaufel über den Zuluftkanal des Ohrtrichters geführt und in den äußeren Gehörgang geleitet. Nach der Trocknung des äußeren Gehörgangs, insbesondere des Recessus meatus acustici externi, kann die feuchte Luft den äußeren Gehörgang über die Einströmöffnung verlassen und wird über den Abluftkanal zu dem Auslass des Ohrtrichters geführt, über welchen Auslass die feuchte Luft aus dem Aufsatz ausgestoßen wird. Innerhalb des Aufsatzes kommt es somit zu keiner oder nur in einem sehr geringen Maße zu einer Vermischung der im Zuluftkanal und im Abluftkanal geführten Luftmassen, was die Effizienz der Trocknung weiter erhöht.

Im Allgemeinen können der Leitschaufelboden oder die Leitschaufelwangen den Ohrtrichter, insbesondere die Innenseite des Ohrtrichters, entweder an keiner Stelle oder über die gesamte Länge des Ohrtrichters oder nur im Bereich der proximalen Öffnung des Ohrtrichters kontaktieren. Es sind auch Ausführungsvarianten denkbar, in denen der Leitschaufelboden oder die Leitschaufelwangen den Ohrtrichter abschnittsweise kontaktiert und abschnittweise von dem Ohrtrichter, nämlich dessen Innenseite, beabstandet ist bzw. sind.

Gemäß einer weiteren bevorzugten Ausführungsform ist es vorgesehen, dass ein Querschnitt des Zuluftkanals in Strömungsrichtung verlaufend abnimmt.

Die Strömungsrichtung ist dabei jene Richtung, in welcher der Luftstrom den Zuluftkanal durchströmt, um in den äußeren Gehörgang zu gelangen. Durch diese Querschnittsverengung des Zuluftkanals, die vorzugsweise kontinuierlich ausgebildet ist, kann die Austrittsgeschwindigkeit, mit welcher der Luftstrom den Aufsatz verlässt, eingestellt und eine für den gewünschten Trocknungseffekt besonders vorteilhafte Austrittsgeschwindigkeit gewählt werden.

Der Aufsatz ist derart dimensioniert, dass der Auslass des Aufsatzes außerhalb des äußeren Gehörgangs zu liegen kommt. Dabei ist der Auslass besonders bevorzugt durch eine Öffnung in einer Gehäusewand des Ohrtrichters gebildet, vorzugsweise in einem dem Verbindungsstutzen unmittelbar vorgeordneten Abschnitt des Ohrtrichters.

Die feuchte Luft, die bereits zur Trocknung verwendet wurde, wird also nicht mehr in den Verbindungsstutzen oder gar in die Vorrichtung eingeleitet, sondern verlässt den Aufsatz bereits im Bereich des Ohrtrichters. Dadurch ergibt sich eine weitere Vereinfachung des Aufbaus des Aufsatzes und auch der Vorrichtung selbst. Insgesamt können durch diese Maßnahme die Herstellungskosten des Aufsatzes niedrig gehalten werden. Durch die Anordnung des Auslasses in dem dem Verbindungsstutzen vorgelagerten Abschnitt des Ohrtrichters kann der Auslass zudem auf besonders einfache Weise gefertigt werden. Dabei ist der Auslass in einem Abschnitt des Ohrtrichters angeordnet, welcher Abschnitt bei bestimmungsgemäßer Verwendung des Aufsatzes zumindest teilweise, vorzugsweise vollständig, auf dem äußeren Gehörgang des Anwenders ragt.

Erfindungsgemäß kann der Aufsatz weiters so ausgestaltet sein, dass
- der Verbindungsstutzen und die Leitschaufel miteinander einstückig ausgebildet sind, und vorzugsweise als Einheit mit dem Ohrtrichter verbindbar sind, oder dass
- der Ohrtrichter und die Leitschaufel miteinander einstückig ausgebildet sind, und vorzugsweise als Einheit mit dem Verbindungsstutzen verbindbar sind, oder dass
- der Verbindungsstutzen, die Leitschaufel und der Ohrtrichter miteinander einstückig ausgebildet sind.

Im Falle der einstückigen Ausbildung von Verbindungsstutzen und Leitschaufel können unterschiedlich ausgebildete Ohrtrichter in Verbindung mit ein und derselben den Verbindungsstutzen und die Leitschaufel umfassenden Einheit verwendet werden, welche Einheit dann als separates Ersatzteil angeboten werden kann. Somit kann der Aufsatz an unterschiedliche Gehörgänge angepasst werden, ohne dafür den Verbindungsstutzen oder die Leitschaufel austauschen zu müssen. Dies ist insbesondere deshalb von Vorteil, da sich die Ausgestaltung des Verbindungsstutzens vor allem nach Größe und Form einer Auslassöffnung für den Luftstrom der Vorrichtung richtet und somit auf die jeweils verwendete Vorrichtung abgestimmt ist. Dadurch kann der Ohrtrichter unabhängig von den übrigen Bestandteilen des Aufsatzes speziell auf die jeweilige Anwendergruppe abgestimmt werden. Beispielsweise können Material, Größe und/oder Form des Ohrtrichters in Abhängigkeit davon gewählt werden, ob der Aufsatz von weilblichen oder männlichen Erwachsenen oder Kindern verwendet werden soll, während die übrigen Bestandteile des Aufsatzes auf die jeweilige Vorrichtung abgestimmt sind, in Verbindung mit welcher der Aufsatz zum Einsatz kommen soll. Somit ist der Ohrtrichter leicht auswechselbar und unabhängig von den anderen Bestandteilen des Aufsatzes nachrüstbar. Die Möglichkeit, den Ohrtrichter nach jeder erfolgten Behandlung des Ohres auszuwechseln, ist darüber hinaus auch aus hygienischer Sicht sehr günstig.

Im Falle der einstückigen Ausbildung von Ohrtrichter und Leitschaufel können sämtliche Teile des Aufsatzes, die bei bestimmungsgemäßer Verwendung des Aufsatzes in Kontakt mit dem äußeren Gehörgang des Anwenders geraten (können), nämlich Ohrtrichter und Leitschaufel, als separates Ersatzteil angeboten und mit dem Verbindungsstutzen verbunden werden. Dies ermöglicht eine besonders hygienische Verwendung des erfindungsgemäßen Aufsatzes, da die den Ohrtrichter und die Leitschaufel umfassende Einheit nach jeder Anwendung getauscht werden kann.

Im Falle der einstückigen Ausbildung von Ohrtrichter, Leitschaufel und Verbindungsstutzen kann der gesamte Aufsatz als separates Ersatzteil angeboten und nach jeder Anwendung ausgetauscht werden. Diese Ausführungsform ist zudem sehr kostengünstig.

Gemäß einer weiteren Ausführungsform ist es vorgesehen, dass der Verbindungsstutzen durch einen Endabschnitt des Ohrtrichters ausgebildet ist.

Insbesondere im Falle einer einstückigen Ausbildung von Ohrtrichter, Leitschaufel und Verbindungsstutzen kann durch den damit verbundenen Wegfall eines zusätzlichen separaten Bauteils der erfindungsgemäße Aufsatz kompakter gestaltet und kostengünstiger hergestellt werden.

Die der Erfindung zu Grunde liegende Aufgabe wird auch gelöst durch ein Ohrentrocknungsgerät umfassend eine Vorrichtung zur Generierung eines Luftstromes, vorzugsweise eines Warmluftstromes, sowie einen erfindungsgemäßen Aufsatz gemäß den vorhergehend beschriebenen Ausführungsformen.

In analoger Weise wird eine weitere, der Erfindung zu Grunde liegende Aufgabe gelöst durch ein Gerät zur pflegenden oder therapeutischen Behandlung des äußeren Gehörgangs eines menschlichen oder tierischen Ohres, umfassend eine Vorrichtung zur Abgabe eines Fluids sowie einen erfindungsgemäßen Aufsatz gemäß einer der vorhergehend beschriebenen Ausführungsformen.

Bei einer bevorzugten Ausführungsform des erfindungsgemäßen Aufsatzes ist es vorgesehen, dass der Verbindungsstutzen dazu eingerichtet ist, den Aufsatz verdrehbar an die Vorrichtung anzubinden.

Die Orientierung des Aufsatzes und somit auch die Richtung, aus der die (Warm-) Luft oder das Fluid in den Gehörgang einströmt, kann somit je nach konkretem Anwendungsfall (Trocknung, Cerumen-Entfernung, Abgabe eines pflegenden oder therapeutischen Fluids in den Gehörgang) gewählt bzw. an die persönlichen Bedürfnisse des Anwenders angepasst werden.

Dabei ist es besonders vorteilhaft, wenn der Verbindungsstutzen an einer äußeren Mantelfläche zumindest eine Vertiefung aufweist, welche Vertiefung mit zumindest einer Rastnase der Vorrichtung in Eingriff bringbar ist, um den Aufsatz in einer bestimmten Drehposition an der Vorrichtung zu arretieren.

Dadurch kann eine bestimmte Drehposition des Aufsatzes, die beispielsweise für die Trocknung des Gehörgangs optimal ist, vorgegeben werden; der Aufsatz kann weiterhin leicht in andere Drehpositionen gebracht werden, verrastet jedoch in der vorgegebenen Drehposition mit der Rastnase der Vorrichtung. Die Rastnase kann etwa an einer Innenseite der Vorrichtung im Bereich der Auslassöffnung der Vorrichtung für den Fluidstrom angeordnet sein.

Besonders bevorzugt sind mehrere Vertiefungen voneinander beabstandet auf der äußeren Mantelfläche des Verbindungsstutzens angeordnet, um den Aufsatz in verschiedenen Drehpositionen an der Vorrichtung arretieren zu können.

So können verschiedene Drehpositionen vorgegeben werden, von denen beispielsweise jeweils eine für einen bestimmten Anwendungsfall (Trocknung, Cerumen-Entfernung, Abgabe eines pflegenden oder therapeutischen Fluids in den Gehörgang) optimal ist.

Bevorzugt weisen die einzelnen Vertiefungen einen Winkelabstand von 180°, vorzugsweise von 90°, besonders bevorzugt von 45°, zueinander auf. Das bedeutet, dass bei einem Winkelabstand von 180° zwei, bei einem Winkelabstand von 90° vier, und bei einem Winkelabstand von 45° acht äquidistante Vertiefungen auf der äußeren Mantelfläche des Verbindungsstutzens angeordnet sein können.

Dadurch kann der Aufsatz in unterschiedlichen, ausgezeichneten Drehstellungen an der Vorrichtung arretiert werden.

Um den (Warm-) Luft- bzw. Fluidstrom noch vorteilhafter in den äußeren Gehörgang des Anwenders einleiten zu können, ist es bei einer anderen bevorzugten Ausführungsform des erfindungsgemäßen Aufsatzes vorgesehen, dass zumindest ein Anströmelement vorgesehen ist, welches Anströmelement von dem Leitschaufelboden abragt, um das aus der Vorrichtung ausströmende, den Aufsatz durchströmende Fluid zu verwirbeln.

Ein solches Anströmelement dient somit als Strömungsstörer, durch welchen die im Wesentlichen anliegende Strömung des Fluids durch den Aufsatz verwirbelt wird; nach Passieren des Anströmelementes führt die Strömung eine drehende Bewegung aus (Vortex). Eine derart durch ein oder mehrere Anströmelemente modifizierte Strömung führt dazu, dass die oben beschriebenen vorteilhaften Trocknungs-, Säuberungs- und Behandlungswirkungen weiter verstärkt werden.

Bevorzugt sind zumindest zwei, vorzugsweise flächig ausgebildete, Anströmelemente vorgesehen, welche einander gegenüberliegend auf dem Leitschaufelboden angeordnet sind. Dabei können die Anströmelemente näherungsweise in Strömungsrichtung des Fluids bzw. in Längsrichtung des Aufsatzes ausgerichtet sein.

Der durch den Verbindungsstutzen in den Aufsatz einströmende Fluidstrom wird durch die Anströmelemente verwirbelt, wobei es durch die paarweise gegenüberliegende Anordnung in dem zwischen den Anströmelementen liegenden Bereich des Aufsatzes zu einer Verdichtung des Fluidstromes kommt, die vorteilhaft für die gewünschten Effekte im Zusammenhang mit der Trocknung, der Cerumen-Entfernung oder der Abgabe eines pflegenden oder therapeutischen Fluids in den Gehörgang ist.

Bei einer anderen bevorzugten Ausführungsform weisen die Anströmelemente die Form eines, vorzugsweise rechtwinkeligen, Dreiecks auf.

Dabei können die Anströmelemente mit einer Kathete des Dreiecks so an dem Leitschaufelboden befestigt sein, sodass die andere Kathete dem proximalen Ende des Leitschaufelbodens zugewandt ist und der durch den Verbindungsstutzen in den Aufsatz einströmende Fluidstrom über die Hypotenuse des Dreiecks in eine turbulente Strömung bzw. eine Verwirbelung gezwungen wird.

Besonders bevorzugt verlaufen Anströmflächen der Anströmelemente, welche Anströmflächen dem Verbindungsstutzen zugewandt sind, schräg zueinander.

Somit sind die Anströmflächen dem aus der Vorrichtung in den Aufsatz eintretenden Fluidstrom zugewandt. Durch Auftreffen des Fluidstroms auf die Anströmflächen kommt es zu einer besonders vorteilhaften Ablenkung des (zuvor näherungsweise an dem Leitschaufelboden anliegenden) Fluidstromes in dem Aufsatz.

Bei einer weiteren bevorzugten Ausführungsform des erfindungsgemäßen Aufsatzes ist es vorgesehen, dass, von dem Verbindungsstutzen aus betrachtet, ein Abstand zwischen gegenüberliegenden Anströmelementen in Längsrichtung des Aufsatzes abnimmt.

Die durch derart ausgebildete Anströmelemente hervorgerufene Fluidströmung weist näherungsweise zwei Wirbelströmungen auf, wobei jeder dieser Wirbel durch eines der Anströmelemente hervorgerufen wird. Durch die Annäherung der paarweise gegenüberliegend angeordneten Anströmelemente in Längsrichtung des Aufsatzes - und somit in Strömungsrichtung des Fluids - kommt es im Gehörgang zu einer für die gewünschten Anwendungen besonders günstigen Strömungsverteilung.

Bei einer anderen bevorzugten Ausführungsform des erfindungsgemäßen Aufsatzes ist der Leitschaufelboden zumindest im Bereich eines zweiten Längsabschnitts des Leitschaufelbodens, vorzugsweise seines zweiten Abschnitts, gegenüber einem ersten Längsabschnitt, vorzugsweise dem ersten Abschnitt, verdreht.

Beispielsweise kann der Leitschaufelboden ähnlich einer geometrischen Regel(hyper)fläche ausgebildet sein, sodass der zweite Längsabschnitt, vorzugsweise ein Scheitelpunkt des zweiten Längsabschnitts, gegenüber dem ersten Längsabschnitt, vorzugsweise einem Scheitelpunkt des ersten Längsabschnitts, entlang einer quer zur Längsachse des Aufsatzes verlaufenden Kurve verschoben ist und/oder die Orientierung des zweiten Längsabschnitts von jener des ersten Längsabschnitts abweicht.

Dadurch wird es möglich, dem in den Gehörgang eingeleiteten Fluidstrom gezielt einen Drall zu verleihen. Die dadurch entstehende Strömungsverteilung des Fluidstromes bewirkt eine besonders effektive Trocknung bzw. Säuberung des Gehörgangs. Auch kann pflegende oder therapeutische Flüssigkeit, die über den Aufsatz in den Gehörgang eingeleitet wird, besser im Gehörgang verteilt werden und so auch an Stellen gelangen, die bei bekannten Behandlungsmethoden nicht behandelt werden können.

Dabei ist es vorteilhaft, wenn die erste Leitschaufelwange und die zweite Leitschaufelwange, insbesondere gegenüberliegende Abschnitte der Leitschaufelwangen, jeweils eine unterschiedliche Höhe aufweisen.

Obgleich die mit den vorstehend als bevorzugt bezeichneten Ausführungsformen einhergehenden vorteilhaften Effekte synergistisch mit der erfindungsgemäß ausgebildeten Leitschaufel zusammenwirken, können die entsprechenden Vorteile auch unabhängig von der speziellen Form der Leitschaufel bzw. des Leitschaufelbodens erreicht werden. Insbesondere sind die folgenden Ausführungsformen vom Erfindungsgedanken mitumfasst:
Aufsatz für eine Vorrichtung zur Generierung eines Luftstromes, vorzugsweise eines Warmluftstromes, oder zur Abgabe eines Fluids, welcher Aufsatz dazu eingerichtet ist, zumindest abschnittsweise in einen äußeren Gehörgang eines menschlichen oder tierischen Ohres eingeführt zu werden, wobei der Aufsatz einen Verbindungsstutzen zur Anbindung an die Vorrichtung, sowie eine zungenartig ausgebildete Leitschaufel mit einem Leitschaufelboden zur Führung eines aus der Vorrichtung ausströmenden Fluidstromes aufweist, und wobei der Verbindungsstutzen dazu eingerichtet ist, den Aufsatz verdrehbar an die Vorrichtung anzubinden, wobei vorzugsweise der Verbindungsstutzen an einer äußeren Mantelfläche zumindest eine Vertiefung aufweist, welche Vertiefung mit zumindest einer Rastnase der Vorrichtung in Eingriff bringbar ist, um den Aufsatz in einer bestimmten Drehposition an der Vorrichtung zu arretieren, wobei vorzugsweise mehrere Vertiefungen voneinander beabstandet auf der äußeren Mantelfläche des Verbindungsstutzens angeordnet sind, um den Aufsatz in verschiedenen Drehpositionen an der Vorrichtung arretieren zu können, wobei vorzugsweise die Vertiefungen einen Winkelabstand von 180°, vorzugsweise von 90°, besonders bevorzugt von 45°, zueinander aufweisen.

Aufsatz für eine Vorrichtung zur Generierung eines Luftstromes, vorzugsweise eines Warmluftstromes, oder zur Abgabe eines Fluids, welcher Aufsatz dazu eingerichtet ist, zumindest abschnittsweise in einen äußeren Gehörgang eines menschlichen oder tierischen Ohres eingeführt zu werden, wobei der Aufsatz einen Verbindungsstutzen zur Anbindung an die Vorrichtung, sowie eine zungenartig ausgebildete Leitschaufel mit einem Leitschaufelboden zur Führung eines aus der Vorrichtung ausströmenden Fluidstromes aufweist, und wobei zumindest ein Anströmelement vorgesehen ist, welches Anströmelement von dem Leitschaufelboden abragt, um das aus der Vorrichtung ausströmende Fluid zu verwirbeln, wobei vorzugsweise zumindest zwei, vorzugsweise flächig ausgebildete, Anströmelemente vorgesehen sind, welche einander gegenüberliegend auf dem Leitschaufelboden angeordnet sind, wobei vorzugsweise die Anströmelemente die Form eines, vorzugsweise rechtwinkeligen, Dreiecks aufweisen, wobei vorzugsweise Anströmflächen der Anströmelemente, welche Anströmflächen dem Verbindungsstutzen zugewandt sind, schräg zueinander verlaufen, wobei vorzugsweise ein Abstand zwischen gegenüberliegenden Anströmelementen, von dem Verbindungsstutzen aus betrachtet, in Längsrichtung des Aufsatzes abnimmt.

Aufsatz für eine Vorrichtung zur Generierung eines Luftstromes, vorzugsweise eines Warmluftstromes, oder zur Abgabe eines Fluids, welcher Aufsatz dazu eingerichtet ist, zumindest abschnittsweise in einen äußeren Gehörgang eines menschlichen oder tierischen Ohres eingeführt zu werden, wobei der Aufsatz einen Verbindungsstutzen zur Anbindung an die Vorrichtung, sowie eine zungenartig ausgebildete Leitschaufel mit einem Leitschaufelboden zur Führung eines aus der Vorrichtung ausströmenden Fluidstromes aufweist, und wobei der Leitschaufelboden zumindest im Bereich eines zweiten Längsabschnitts gegenüber einem ersten Längsabschnitt verdreht ist, wobei vorzugsweise die erste Leitschaufelwange und die zweite Leitschaufelwange jeweils eine unterschiedliche Höhe aufweisen.

### KURZE BESCHREIBUNG DER FIGUREN

Die Erfindung wird nun anhand von Ausführungsbeispielen näher erläutert. Die Figuren sind beispielhaft und sollen den Erfindungsgedanken zwar darlegen, ihn aber keinesfalls einengen oder gar abschließend wiedergeben. Dabei zeigt:
- Fig. 1a: einen erfindungsgemäßen Aufsatz in einer ersten Ausführungsform
- Fig. 1b: einen Ohrtrichter der ersten Ausführungsform des erfindungsgemäßen Aufsatzes
- Fig. 2a: die erste Ausführungsform des Aufsatzes mit Ohrtrichter in einer ersten perspektivischen Ansicht
- Fig. 2b: die erste Ausführungsform des Aufsatzes mit Ohrtrichter in einer zweiten perspektivischen Ansicht
- Fig. 3a: einen Längsschnitt durch den Aufsatz aus Fig. 2a und 2b, der an einer Vorrichtung zur Generierung eines Luftstromes oder zur Abgabe eines Fluids befestigt ist
- Fig. 3b: einen Längsschnitt durch den Aufsatz aus Fig. 2a und 2b
- Fig. 4a: einen Querschnitt des Aufsatzes aus Fig. 3b gemäß A-A
- Fig. 4b: einen Querschnitt des Aufsatzes aus Fig. 3b gemäß B-B
- Fig. 4c: einen Querschnitt des Aufsatzes aus Fig. 3b gemäß C-C
- Fig. 5a: ein erfindungsgemäßes Ohrentrocknungsgerät umfassend den Aufsatz gemäß der ersten Ausführungsform ohne Ohrtrichter
- Fig. 5b: das erfindungsgemäße Ohrentrocknungsgerät umfassend den Aufsatz gemäß der ersten Ausführungsform mit Ohrtrichter
- Fig. 5c: das erfindungsgemäße Ohrentrocknungsgerät mit einer Schutzkappe
- Fig. 6a: einen ersten durch den erfindungsgemäßen Aufsatz hervorgerufenen Strömungsverlauf in einem äußeren Gehörgang
- Fig. 6b: einen zweiten durch den erfindungsgemäßen Aufsatz hervorgerufenen Strömungsverlauf in einem äußeren Gehörgang
- Fig. 6c: einen dritten durch den erfindungsgemäßen Aufsatz hervorgerufenen Strömungsverlauf in einem äußeren Gehörgang
- Fig. 7a: eine Liniendarstellung der Fig. 1a
- Fig. 7b: eine Liniendarstellung der Fig. 1b
- Fig. 7c: eine Liniendarstellung der Fig. 2a
- Fig. 7d: eine Liniendarstellung der Fig. 2b
- Fig. 8a: eine perspektivische Ansicht einer Ausführungsform des erfindungsgemäßen Aufsatzes mit Vertiefungen
- Fig. 8b: den Aufsatz aus Fig. 8a in einer Seitenansicht
- Fig. 8c: den Aufsatz aus Fig. 8a in Längsrichtung des Aufsatzes betrachtet
- Fig. 8d: Detail F aus Fig. 8c
- Fig. 9a: eine Vorrichtung zur Abgabe eines Fluids im Bereich einer Auslassöffnung in einer Schnittdarstellung
- Fig. 9b: Detail G aus Fig. 9a
- Fig. 10a: eine Ausführungsform des erfindungsgemäßen Aufsatzes vor der Anbindung an eine Vorrichtung zur Abgabe eines Fluids
- Fig. 10b: eine an eine Vorrichtung zur Abgabe eines Fluids angebundene Ausführungsform des erfindungsgemäßen Aufsatzes
- Fig. 11a: eine Ausführungsform des erfindungsgemäßen Aufsatzes mit Anströmelementen in Längsrichtung des Aufsatzes betrachtet
- Fig. 11b: den Aufsatz aus Fig. 11a in einer perspektivischen Darstellung
- Fig. 12a: eine Ausführungsform des erfindungsgemäßen Aufsatzes mit asymmetrischer Leitschaufel in Längsrichtung des Aufsatzes betrachtet
- Fig. 12b: den Aufsatz aus Fig. 12a in entgegengesetzter Richtung betrachtet
- Fig. 13a: eine erste perspektivische Ansicht des Aufsatzes aus Fig. 12a
- Fig. 13b: eine zweite perspektivische Ansicht des Aufsatzes aus Fig. 12a
- Fig. 13c: eine dritte perspektivische Ansicht des Aufsatzes aus Fig. 12a

### WEGE ZUR AUSFÜHRUNG DER ERFINDUNG

In der nachfolgenden Beschreibung bezeichnet der Begriff "Längsschnitt" einen Schnitt durch einen erfindungsgemäßen Aufsatz 1, bei dem die Schnittebene parallel zur Zeichenebene der Figuren verläuft und eine Längsachse 12 des Aufsatzes 1 in der Schnittebene liegt. Des Weiteren bezeichnet der Begriff "Querschnitt" einen Schnitt durch den Aufsatz 1, bei dem die Schnittebene orthogonal zu dem Längsschnitt verläuft.

Grundsätzlich existiert eine Vielzahl denkbarer Ausführungsformen des erfindungsgemäßen Aufsatzes, wobei all diesen Ausführungsformen gemein ist, dass ein Luftstrom durch eine Leitschaufel verwirbelt wird und/oder aufgrund der Leitschaufelgeometrie an Seitenwänden eines äußeren Gehörgangs 3 eines Anwenders reflektiert wird, um im Inneren des äußeren Gehörgangs 3 einen optimalen Trocknungseffekt zu bewirken.

In einer ersten Ausführungsform umfasst der erfindungsgemäße Aufsatz 1 einen Verbindungsstutzen 4 sowie eine zungenartig ausgebildete Leitschaufel 5, und kann darüber hinaus optional einen Ohrtrichter 18 umfassen, wobei der Verbindungsstutzen 4 und die Leitschaufel 5 miteinander einstückig ausgebildet und lösbar mit dem Ohrtrichter 18 verbindbar sind. Diese Ausführungsform zeichnet sich unter anderem dadurch aus, dass der Ohrtrichter 18 abnehmbar und der Aufsatz 1 mit oder ohne Ohrtrichter 18 nutzbar ist. Der Ohrtrichter 18, der bei dieser Ausführungsform nicht zwingend vorgesehen ist, ist - wenn vorhanden - leicht auswechselbar und unabhängig von dem Verbindungsstutzen 4 und der Leitschaufel 5 nachrüstbar. Die Möglichkeit, den Ohrtrichter 18 nach jedem Trocknungsvorgang auszuwechseln, ist darüber hinaus auch aus hygienischer Sicht sehr günstig.

In einer zweiten Ausführungsform umfasst der erfindungsgemäße Aufsatz ebenfalls den Verbindungsstutzen, die Leitschaufel und den Ohrtrichter, wobei der Ohrtrichter und die Leitschaufel bei dieser Ausführungsform miteinander einstückig ausgebildet sind und die Einheit aus Ohrtrichter und Leitschaufel mit dem Verbindungsstutzen verbindbar ist. Dabei können sämtliche Teile des Aufsatzes, die bei bestimmungsgemäßer Verwendung des Aufsatzes in Kontakt mit dem äußeren Gehörgang 3 des Anwenders geraten (können), nämlich Ohrtrichter und Leitschaufel, als separates Ersatzteil angeboten und mit dem Verbindungsstutzen verbunden werden. Dies ermöglicht eine besonders hygienische Verwendung des erfindungsgemäßen Aufsatzes, da die den Ohrtrichter und die Leitschaufel umfassende Einheit nach jeder Anwendung getauscht werden kann.

In einer dritten Ausführungsform umfasst der erfindungsgemäße Aufsatz ebenfalls den Verbindungsstutzen, die Leitschaufel und den Ohrtrichter, wobei Verbindungsstutzen, Leitschaufel und Ohrtrichter bei dieser Ausführungsform miteinander einstückig ausgebildet sind. Dadurch kann der gesamte Aufsatz als separates Ersatzteil angeboten und nach jeder Anwendung ausgetauscht werden. Daraus ergibt sich eine besonders einfache und hygienische Handhabung des Aufsatzes gemäß der dritten Ausführungsform. Des Weiteren kann der Aufsatz durch seine einstückige Ausbildung besonders einfach und dadurch kostengünstig hergestellt werden.

Ebenfalls vom Erfindungsgedanken mitumfasst sind Ausführungsformen des Aufsatzes, bei denen der Verbindungsstutzen, die Leitschaufel und der Ohrtrichter jeweils als separates Bauteil ausgebildet sind.

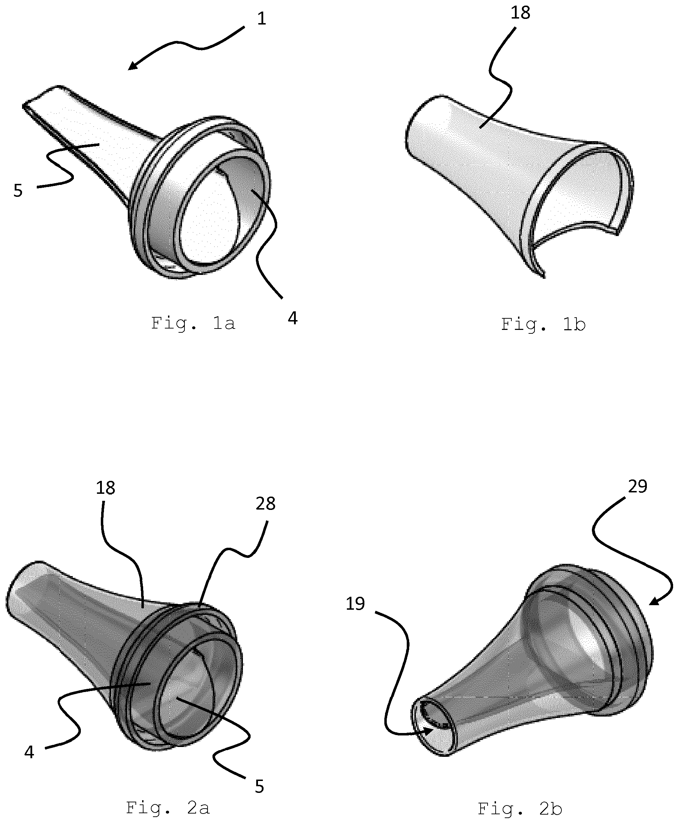

Die erste Ausführungsform des erfindungsgemäßen Aufsatzes wird im Folgenden anhand der Figuren 1 bis 6 näher beschrieben; die zweite und dritte Ausführungsform ist in den Figuren 1 bis 6 hingegen nicht abgebildet.

Fig. 1a ist eine perspektivische Darstellung dieser Ausführungsform des erfindungsgemäßen Aufsatzes 1 ohne Ohrtrichter, welcher bei dieser Ausführungsform optional ist. Der Aufsatz 1 umfasst den Verbindungsstutzen 4 zur Anbindung des Aufsatzes 1 an eine Vorrichtung 2 zur Generierung eines Luftstroms, vorzugsweise eines Warmluftstroms, bzw. zur Abgabe eines Fluids. Der Verbindungsstutzen 4 bildet dabei jenen Abschnitt des Aufsatzes 1 aus, der dafür sorgt, dass der Aufsatz 1 an der Vorrichtung 2 hält.

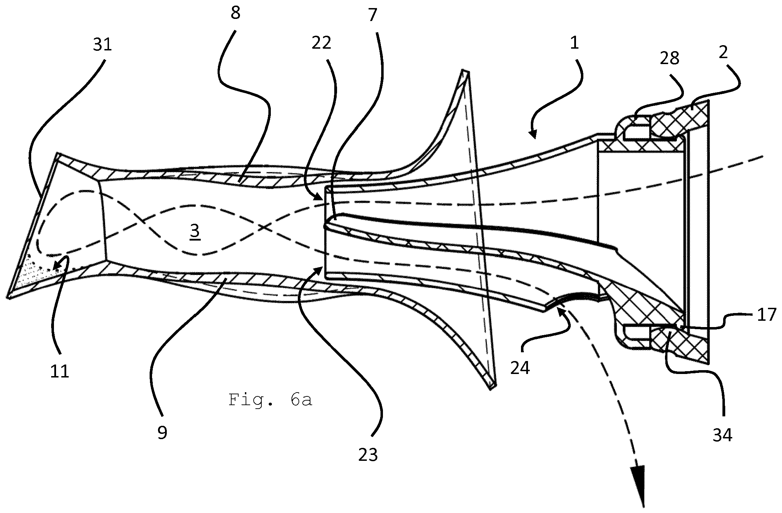

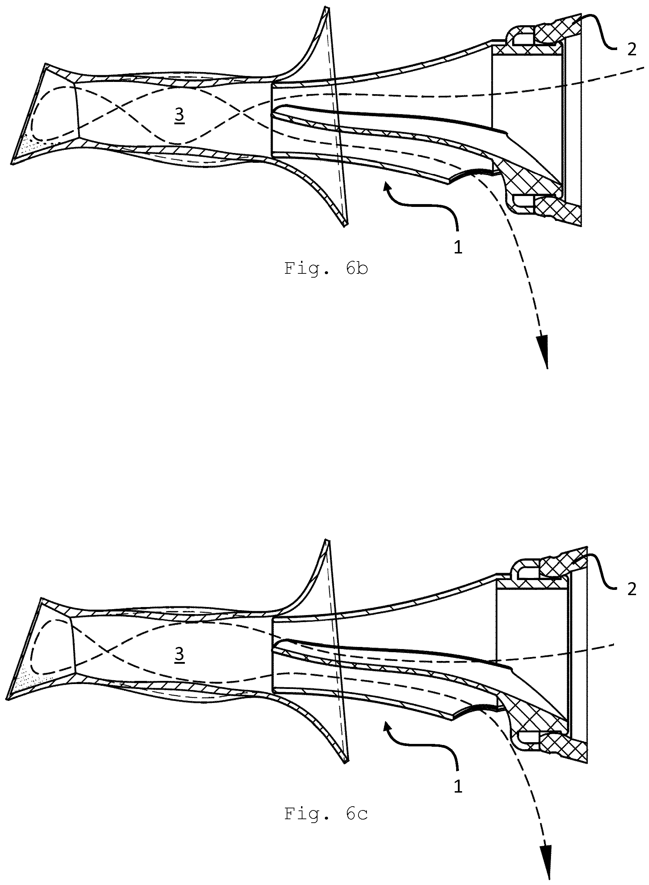

Weiters umfasst der Aufsatz 1 die zungenartig ausgebildete Leitschaufel 5, welche in einen äußeren Gehörgang 3 eines Tieres oder Menschen (im Folgenden Anwender genannt) zwecks Trocknung des Gehörgangs 3 eingeführt werden kann (siehe Fig. 6). Die Leitschaufel 5 ermöglicht eine kontrollierte Führung des Luftstromes sowie dessen kontrollierte Abgabe an den äußeren Gehörgang 3. Die Leitschaufel 5 weist zur Führung des Luftstromes einen Leitschaufelboden 25 auf und kann außerdem Leitschaufelwangen 26 umfassen, um einen Leitkanal oder einen Teil eines Leitkanals für den Luftstrom auszubilden.

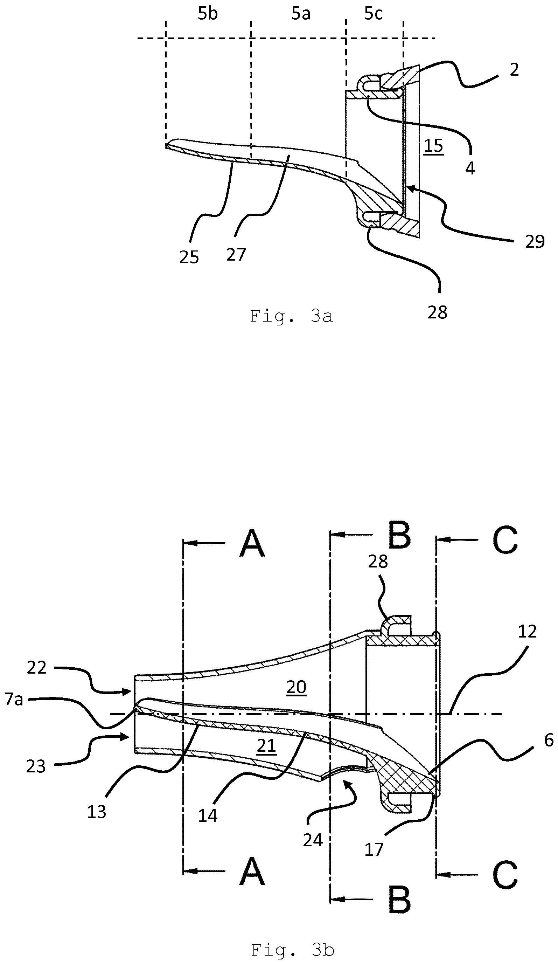

Fig. 3a zeigt einen Längsschnitt des erfindungsgemäßen Aufsatzes 1 gemäß der ersten Ausführungsform ohne Ohrtrichter, welcher Längsschnitt durch den Verbindungsstutzen 4 und die Leitschaufel 5 verläuft. Fig. 3b zeigt den Aufsatz 1 aus Fig. 3a mit Ohrtrichter 18, wobei die in Fig. 3a und 3b gezeigten Aufsätze 1 bis auf den Ohrtrichter 18 identisch ausgebildet sind.

Der im Wesentlichen hülsenförmige Verbindungsstutzen 4 dient der Anbindung des Aufsatzes 1 an die Vorrichtung 2 und ermöglicht ein Einströmen des Luftstromes aus der Vorrichtung 2 in den Aufsatz 1. Die Geometrie des Verbindungsstutzens 4 richtet sich dabei nach der Geometrie einer Auslassöffnung 15 der Vorrichtung 2, durch welche Auslassöffnung 15 der Luftstrom aus der Vorrichtung 2 ausströmt. Die zungenartig ausgebildete Leitschaufel 5 steht im gezeigten Ausführungsbeispiel von dem Verbindungsstutzen 4 ab und lenkt den Luftstrom in den äußeren Gehörgang 3 des Anwenders (siehe Fig. 6).

Um den in Fig. 3a abgebildeten Aufsatz 1 bestimmungsgemäß zur Trocknung des äußeren Gehörgangs 3 verwenden zu können, kann dieser mit der Leitschaufel 5 - entweder abschnittsweise oder vollständig - in den äußeren Gehörgang 3 des Anwenders eingeführt werden.

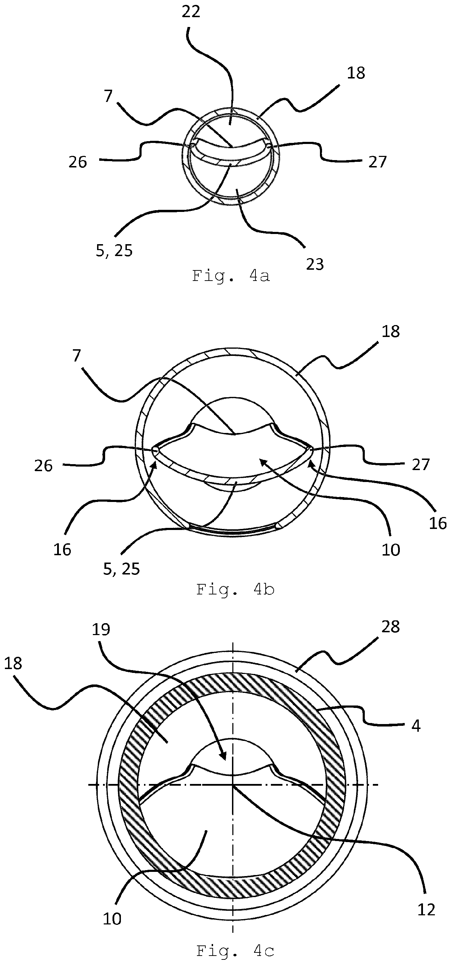

Aus Fig. 3a und 3b ist deutlich ersichtlich, dass die Leitschaufel 5 derart angeordnet ist, dass der Leitschaufelboden 25 die Längsachse 12 des Aufsatzes 1 schneidet. Die Längsachse 12 des Aufsatzes 1 verläuft durch einen Mittelpunkt eines normal auf den in Fig. 3a und 3b gezeigten Längsschnitt stehenden Querschnittes des Verbindungsstutzens 4. Der Querschnitt des Verbindungsstutzens 4 bezieht sich dabei ausschließlich auf den Verbindungsstutzen 4 selbst - insbesondere also nicht auf etwaige innerhalb des Verbindungsstutzens 4 angeordnete Abschnitte der Leitschaufel 5. Der Querschnitt des Verbindungsstutzens 4, durch dessen Mittelpunkt sich die Längsachse 12 erstreckt, ist in Fig. 4c zu sehen.

Durch diese Anordnung der Leitschaufel 5 wird der Luftstrom derart in den äußeren Gehörgang 3 des Anwenders eingeleitet, sodass sich im äußeren Gehörgang 3 ein für den gewünschten Trocknungseffekt besonders vorteilhaftes Strömungsfeld einstellt. Zudem kann dadurch sichergestellt werden, dass der aus dem Aufsatz 1 austretende Luftstrom nicht direkt auf das Trommelfell 31 des Anwenders geleitet wird. Der Luftstrom wird nämlich, wenn er über ein proximales Ende 7a des Leitschaufelbodens 25 strömt und den Aufsatz 1 verlässt, verwirbelt und/oder derart in den äußeren Gehörgang 3 eingeleitet, dass der Luftstrom an den Seitenwänden des äußeren Gehörgangs 3 reflektiert wird, um im Inneren des äußeren Gehörgangs 3 einen optimalen Trocknungseffekt zu bewirken. Vorzugsweise schneidet der Leitschaufelboden 25 die Längsachse 12 in einem proximalen - weil dem Trommelfell 31 des Anwenders nächstgelegenen - Endbereich des Aufsatzes 1.

Ebenfalls aus Fig. 3a und 3b ersichtlich ist, dass der Leitschaufelboden 25 einen gekrümmten Längsschnitt aufweist, welcher normal auf den in den Figuren 4a und 4b abgebildeten Querschnitt des Leitschaufelbodens 25 steht. Der Leitschaufelboden 25 eines vom Verbindungsstutzen 4 abstehenden ersten Abschnitts 5a der Leitschaufel 5 weist einen Längsquerschnittsverlauf auf, der in eine erste Richtung gekrümmt ist, und der Leitschaufelboden 25 eines vom Verbindungsstutzen 4 abstehenden zweiten Abschnitts 5b der Leitschaufel 5 weist einen Längsquerschnittsverlauf auf, der in eine zweite Richtung gekrümmt ist, wobei die beiden gekrümmten Abschnitte 5a,5b zu einander entgegengesetzt gekrümmt verlaufen. In dem in Fig. 3a und 3b gezeigten Ausführungsbeispiel weist der zweite Abschnitt 5b ein proximales Ende 7 der Leitschaufel 5 auf.

Somit weist die Leitschaufel 5 zumindest einen ersten Scheitelpunkt 13 und einen zweiten Scheitelpunkt 14 auf. Durch diesen speziellen Verlauf der Leitschaufel 5 wird eine besonders günstige Strömungsverteilung des Luftstromes innerhalb des äußeren Gehörganges 3 erzielt. Bei dieser Ausführungsform der Erfindung konnten besonders gute Ergebnisse bei der Trocknung erzielt werden, insbesondere im Vergleich mit Aufsätzen umfassend eine Leitschaufel 5 mit geradem Längsquerschnittsverlauf. Besonders vorteilhaft ist es, wenn - von dem Verbindungsstutzen 4 aus betrachtet - die Steigung einer an den Leitschaufelboden 25 angelegten Tangente im ersten Abschnitt 5a des Leitschaufelbodens 25 mit dem Abstand von dem Verbindungsstutzen 4 abnimmt und im zweiten Abschnitt 5b des Leitschaufelbodens 25 mit dem Abstand von dem Verbindungsstutzen 4 zunimmt.

Die Leitschaufel 5 weist einen im Verbindungsstutzen 4 verlaufenden Abschnitt 5c auf, wobei der Abschnitt 5c ein distales Ende 6 der Leitschaufel 5 aufweist. Der Abschnitt 5c schließt an den Abschnitt 5a der Leitschaufel 5 an. Ein Strömungsquerschnitt für aus der Vorrichtung 2 zur Erzeugung eines Luftstroms in den Aufsatz 1 strömende Luft ist durch den im Verbindungsstutzen 4 verlaufenden Abschnitt 5c der Leitschaufel 5 und den Verbindungsstutzen 4 begrenzt. Dadurch wird jener Anteil des gesamten Luftstromes einstellbar, welcher ungehindert von dem Verbindungsstutzen 4 auf die Leitschaufel 5 übergehen soll. Insbesondere kann der Luftstrom dadurch auch einer Strömungsquerschnittsverengung unterzogen werden, welche Strömungsquerschnittsverengung bei Aufsätzen für Kinder beispielsweise weniger drastisch ausfallen kann als bei Aufsätzen für Erwachsene. Der im Verbindungsstutzen 4 verlaufende Abschnitt 5c der Leitschaufel 5 ermöglicht es außerdem, den aus der Vorrichtung 2 zur Erzeugung eines Luftstromes in den Aufsatz 1 einströmenden Luftstrom bereits im Bereich des Verbindungsstutzens 4 aufzunehmen, sodass der Luftstrom bereits unmittelbar mit Eintritt in den Aufsatz 1 entsprechend gelenkt werden kann.

Von längsseitigen Rändern 16 des Leitschaufelbodens 25 stehen zwei Leitschaufelwangen, nämlich eine erste Leitschaufelwange 26 und eine zweite Leitschaufelwange 27 von dem Leitschaufelboden 25, ab, um gemeinsam mit dem Leitschaufelboden 25 einen Leitkanal 10 oder einen Teil eines Leitkanals 10 für den Luftstrom auszubilden. Leitschaufelboden 25 und Leitschaufelwangen 26, 27 bilden dann einen Teil des Leitkanals 10 aus, wenn die Leitschaufelwangen 26, 27 nur in einem Längsabschnitt der Leitschaufel 5, nicht aber über deren gesamte Länge von dem Leitschaufelboden 25 abstehen. Der durch Leitschaufelboden 25 und Leitschaufelwangen 26, 27 ausgebildete Leitkanal 10 für den Luftstrom ist durch jenes Volumen ausgebildet, das von Leitschaufelboden 25, der ersten Leitschaufelwange 26 einerseits, und der zweiten Leitschaufelwange 27 andererseits begrenzt ist. Durch den Leitkanal 10 kann die gezielte und kontrollierte Führung des Luftstromes innerhalb des Aufsatzes 1 sowie die kontrollierte Abgabe des Luftstromes an den äußeren Gehörgang 3 noch weiter verbessert werden. Auch können - bei Ausführungsformen mit Ohrtrichter, etwa im Falle der ersten Ausführungsform des Aufsatzes 1 mit Ohrtrichter 18 gemäß Fig. 3b - durch den Einsatz der Leitschaufelwangen 26,27 etwaige ungewollte Reflexionen des Luftstromes an einer Innenwand des Ohrtrichters 18 vermieden werden.

Fig. 4a zeigt den Querschnitt des erfindungsgemäßen Aufsatzes 1 gemäß Schnittlinie A-A aus Fig. 3b. Fig. 4b zeigt den Querschnitt gemäß Schnittlinie B-B aus Fig. 3b. Der Schnitt verläuft in beiden Fällen jeweils durch den Ohrtrichter 18 sowie durch den Leitschaufelboden 25 der Leitschaufel 5. Fig. 4c zeigt den Querschnitt des Verbindungsstutzens 4 gemäß Schnittlinie C-C aus Fig. 3b.

Wie in den Figuren 4a, 4b zu sehen ist, weist der Leitschaufelboden 25 im gezeigten Ausführungsbeispiel ein, vorzugsweise U-förmig, gekrümmtes Querschnittsprofil auf. Dabei ist der Querschnitt des Leitschaufelbodens 25 über die gesamte Länge der Leitschaufel 5 hinweg, leicht U-förmig, gekrümmt. Die Krümmung kann dabei von einem Ende der Leitschaufel 5 zu einem anderen Ende der Leitschaufel 5 hin zu- oder abnehmen. Es kann auch vorgesehen sein, dass der Querschnitt des Leitschaufelbodens 25 nicht über die gesamte Länge der Leitschaufel 5 hinweg, sondern nur in einem Längsabschnitt der Leitschaufel 5 gekrümmt ist. Auch andere als U-förmige Krümmungen des Querschnittsprofils des Leitschaufelbodens 25 sind denkbar. Zwar zeigen die Figuren 4a und 4b Querschnitte des Aufsatzes 1 mit Ohrtrichter 18 gemäß Fig. 3b; da jedoch der in Fig. 3b gezeigte Aufsatz 1 abgesehen von dem Ohrtrichter 18 mit dem in Figur 3a gezeigten Aufsatz 1 identisch ist, treffen die getätigten Aussagen bezüglich des gekrümmten Querschnittsprofils des Leitschaufelbodens 25 ebenso auf die erste Ausführungsform des Aufsatzes 1 ohne Ohrtrichter 18 gemäß Fig. 3a zu.

Der Aufsatz 1 gemäß der ersten Ausführungsform kann entweder ohne oder mit Ohrtrichter 18 in den äußeren Gehörgang 3 eingeführt werden, um bestimmungsgemäß zur Trocknung des äußeren Gehörgangs 3 verwendet zu werden.

Durch lösbares Verbinden des in Fig. 1a und 3a gezeigten Aufsatzes 1 mit dem in Fig. 1b perspektivisch dargestellten Ohrtrichter 18 erhält man den in den Fig. 2a, 2b und 3b dargestellten Aufsatz 1, nämlich den Aufsatz 1 gemäß erster Ausführungsform mit Ohrtrichter 18. Dazu kann der Ohrtrichter 18 auf den Verbindungsstutzen 4 aufgeschoben - oder auf andere Weise mit diesem oder der Leitschaufel verbunden - werden. Bei anderen Ausführungsformen des Aufsatzes 1, etwa bei der oben beschriebenen dritten Ausführungsform, kann der Verbindungsstutzen 4 durch einen Endabschnitt, vorzugsweise einen distalen - weil von dem Trommelfell 31 des Anwenders entfernt gelegenen - Endabschnitt des Ohrtrichters 18 ausgebildet sein.

Im Falle der ersten Ausführungsform des Aufsatzes 1 mit Ohrtrichter 18 ist der Ohrtrichter 18 lösbar mit dem Verbindungsstutzen 4 verbindbar und ragt von diesem in einer Weise ab, sodass die Leitschaufel 5 ihrer gesamten Länge nach von dem Ohrtrichter 18 und dem daran anschließenden Verbindungsstutzen 4 umhüllt ist. Es kann auch vorgesehen sein, dass Ohrtrichter 18 und Verbindungsstutzen 4 nur einen Längsabschnitt der Leitschaufel 5 umhüllen, sodass die Leitschaufel 5 über den Ohrtrichter 18 hinaus ragt. Der in Fig. 1b gezeigte Ohrtrichter 18 ist im vorliegenden Ausführungsbeispiel bis auf einen Auslass 24 rotationssymmetrisch, wobei die Längsachse 12 des Aufsatzes 1 die entsprechende Rotationsachse des Ohrtrichters 18 bildet.

Aus Fig. 2a, 2b, 3b, 4a, 4b und 4c ist gut ersichtlich, dass sich der Querschnitt des Ohrtrichters 18 von dem Verbindungsstutzen 4 hin zu einer proximalen Öffnung 19 des Ohrtrichters 18, mittels welcher der Ohrtrichter 18 bei bestimmungsgemäßer Verwendung des Aufsatzes 1 in den Gehörgang 3 eingeführt ist, verringert. Diese Querschnittsverringerung macht es möglich, die Einführtiefe des Aufsatzes 1 in den äußeren Gehörgang 3 festzulegen. Damit wird es beispielsweise möglich, den Aufsatz 1 bis auf etwa 15 mm, vorzugsweise bis auf etwa 10 mm, an das Trommelfell 31 des Anwenders heranzuführen.

Das proximale Ende 7 der Leitschaufel 5, bzw. das proximales Ende 7a des Leitschaufelbodens 25, ist im Wesentlichen bündig mit der proximalen Öffnung 19 des Ohrtrichters 18 angeordnet. Demnach befindet sich das proximale Ende 7a des Leitschaufelbodens 25 innerhalb der proximalen Öffnung 19 des Ohrtrichters 18 und unterteilt diese in eine Ausströmöffnung 22 für aus dem Aufsatz in den äußeren Gehörgang 3 strömende Luft, sowie eine Einströmöffnung 23, durch welche Einströmöffnung 23 Luft aus dem äußeren Gehörgang 3 wieder in den Aufsatz 1 strömen kann.

Bei Ausführungsformen des Aufsatzes mit Ohrtrichter, etwa im Falle der ersten Ausführungsform des Aufsatzes 1 mit Ohrtrichter 18, bei denen die Leitschaufel 5 von dem Verbindungsstutzen 4 des Aufsatzes 1 abragt oder daran anschließt, verläuft die Leitschaufel 5 im Wesentlichen über die gesamte Länge des Ohrtrichters 18. Dadurch kann der zur Trocknung dienende Luftstrom über die gesamte Länge des Ohrtrichter 18 geführt und sehr kontrolliert in den äußeren Gehörgang abgegeben werden, ohne eine Verletzung des äußeren Gehörganges 3 und/oder des Trommelfelles zu riskieren.

Um die Effizienz der Trocknung noch weiter zu steigern, kann die Ausströmöffnung 22 kleiner als die Einströmöffnung 23 sein. Beispielsweise kann die Ausströmöffnung 22 ein Drittel der proximalen Öffnung 19 und die Einströmöffnung 23 zwei Drittel der proximalen Öffnung 19 einnehmen.

Da die proximale Öffnung 19 des Ohrtrichters 18 üblicherweise kleiner ist als eine distale Öffnung 29 des Verbindungsstutzens 4, wird die Trocknung durch den Luftstrom durch eine Erhöhung der Austrittsgeschwindigkeit des Luftstromes aus dem Aufsatz 1 weiter unterstützt und ein besonders rasches und vollständiges Entweichen der feuchten Luft aus dem Gehörgang 3 sichergestellt. Über die distale Öffnung des Verbindungsstutzens 4 strömt der Luftstrom aus der Vorrichtung 2 in den Aufsatz 1 ein.

Bei Ausführungsformen des Aufsatzes mit Ohrtrichter, etwa im Falle der ersten Ausführungsform des Aufsatzes 1 mit Ohrtrichter 18, ist ein Innenvolumen des Ohrtrichters 18 durch den Leitschaufelboden 25, und gegebenenfalls die erste Leitschaufelwange 26 und die zweite Leitschaufelwange 27, in einen Zuluftkanal 20 und einen Abluftkanal 21 unterteilt. Dazu können die längsseitigen Ränder 16 des Leitschaufelbodens 25 oder - falls vorhanden - die daran anschließenden Leitschaufelwangen 26, 27 den Ohrtrichter 18 entweder entlang ihrer gesamten Länge oder nur abschnittsweise direkt kontaktieren, oder aber von dem Ohrtrichter 18 geringfügig beabstandet sein. Im Falle einer Kontaktierung über die gesamte Länge der Leitschaufel 5 kann eine fluidische Isolierung zwischen Zuluftkanal 20 und Abluftkanal 21 erreicht werden. Im Falle einer nur teilweisen Kontaktierung oder einer Beabstandung zwischen Leitschaufel 5 und Ohrtrichter 18 kann zwar keine vollständige Isolierung garantiert, aber immerhin sichergestellt werden, dass eine Vermischung der im Zuluftkanal 20 geführten Luft mit der im Abluftkanal 21 geführten Luft weitestgehend unterbunden wird.

Der Zuluftkanal 20 verbindet die distale Öffnung 29 des Verbindungsstutzens 4 mit der Ausströmöffnung 22. Der Abluftkanal 21 verbindet die Einströmöffnung 23 des Ohrtrichters 18 mit dem Auslass 24 des Aufsatzes 1, welcher an einer Unterseite des Ohrtrichters 18 als Ausnehmung in dessen Gehäuse ausgebildet ist. Demnach ermöglicht der Zuluftkanal 20 ein Einströmen des durch die Vorrichtung 2 erzeugten Luftstromes in den äußeren Gehörgang 3 des Anwenders, und der Abluftkanal 21 ein Zurückströmen der zur Trocknung des äußeren Gehörgangs 3 verwendeten Luft aus dem äußeren Gehörgang 3 in die Atmosphäre.

Um zu verhindern, dass sich die Leitschaufel 5 während der bestimmungsgemäßen Verwendung des erfindungsgemäßen Aufsatzes 1 verformt, wenn der Aufsatz 1 in den äußeren Gehörgang 3 eingeführt wird bzw. ist, kann es vorgesehen sein, dass der Ohrtrichter 18 im Vergleich zum Verbindungsstutzen 4 und/oder zur Leitschaufel 5 starr ausgebildet ist, vorzugsweise weniger flexibel als der Verbindungsstutzen 4 und/oder die Zunge 5 ausgebildet ist. Durch diese Maßnahme schützt der Ohrtrichter 18 insbesondere die Leitschaufel 5 vor einer etwaigen Verformung. Beispielsweise kann der Ohrtrichter 18 aus Polyethylen oder aus Polypropylen gefertigt sein, während Verbindungsstutzen 4 und Leitschaufel 5 aus Thermoplastischem Elastomer oder aus Silikon gefertigt sein kann.

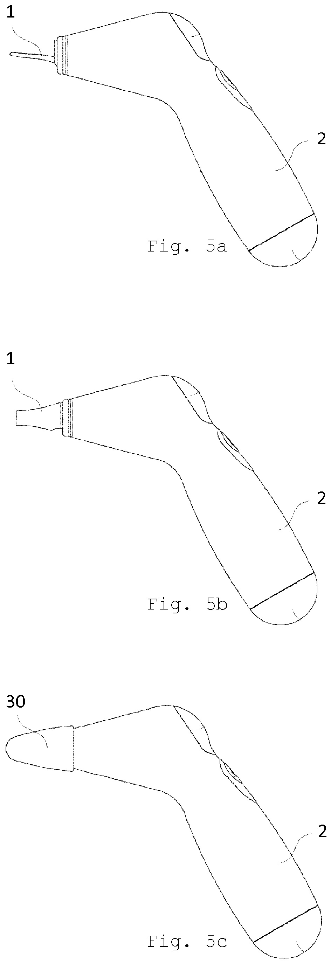

Fig. 3a zeigt den Aufsatz 1 in einer Anbindungsposition, in welcher der Aufsatz 1 an die Vorrichtung 2 (siehe auch Fig. 5a, 5b und 5c) angebunden ist.

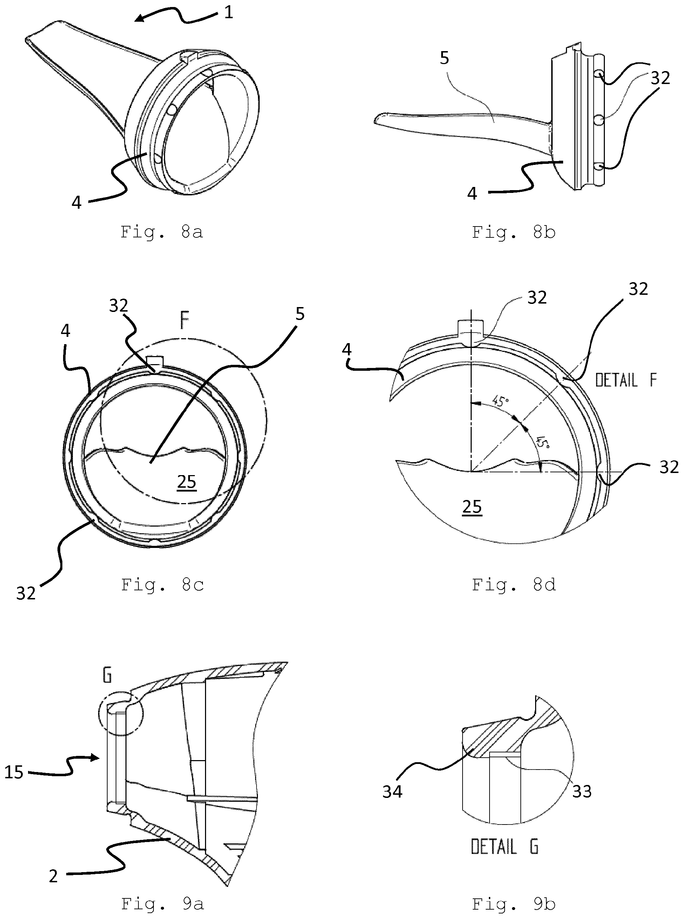

Zum Zwecke der Anbindung ist der Aufsatz 1 mit dem Verbindungsstutzen 4 abschnittweise in die Auslassöffnung 15 für den Luftstrom der Vorrichtung 2 eingeschoben. Ein Rastvorsprung 17 an einem vorrichtungsseitigen Endabschnitt des Verbindungsstutzens 4 steht in Eingriff mit einem Rückhalteabschnitt 34 der Vorrichtung 2 und verhindert so eine unabsichtliche Lösung der Verbindung zwischen der Vorrichtung 2 und dem Aufsatz 1 (siehe beispielsweise Fig. 6a oder 9b. Grundsätzlich sind jedoch viele verschiedene Arten der Anbindung des Aufsatzes 1 an die Vorrichtung 2 denkbar. Der aus der Vorrichtung 2 austretende Luft- oder Fluidstrom durchströmt somit zunächst den Verbindungsstutzen 4 des Aufsatzes 1, wenn er in den Aufsatz 1 einströmt. Dies ist auch dann der Fall, wenn der Verbindungsstutzen 4 nicht in die Auslassöffnung 15 eingeschoben ist sondern auf andere Weise mit der Vorrichtung 2 verbunden, beispielsweise auf diese aufgeschoben, ist.

Ein den Umfang des Verbindungsstutzens 4, zumindest abschnittsweise, umlaufend angeordneter Abstandhalter 28 steht an einem Gehäuse der Vorrichtung 2 an und sorgt so dafür, dass der Verbindungsstutzen 4 nicht tiefer als vorgesehen in die Auslassöffnung 15 eingeschoben werden kann. Bei Ausführungsformen des Aufsatzes mit Ohrtrichter, etwa im Falle der ersten Ausführungsform mit Ohrtrichter 18 gemäß Fig. 3b, kann der Ohrtrichter 18 zum Zwecke der Verbindung auf den Verbindungsstutzen 4 aufgeschoben sein und an dem Abstandhalter 28 anstehen. Hier sind Verbindungsstutzen 4 und Ohrtrichter 18 durch Kraftschluss miteinander verbunden, wobei auch andere Möglichkeiten der Verbindung bestehen - etwa mittels ineinandergreifender Rastnuten und Rastvorsprüngen.

Fig. 5a zeigt ein erfindungsgemäßes Ohrentrocknungsgerät, umfassend die Vorrichtung 2 zur Generierung eines Luftstromes sowie den erfindungsgemäßen Aufsatz 1 gemäß der ersten Ausführungsform des erfindungsgemäßen Aufsatzes, wobei der Ohrtrichter 18 von der einstückig ausgebildeten Einheit aus Verbindungsstutzen 4 und Leitschaufel 5 abgenommen wurde und daher nicht zu sehen ist.

Die Vorrichtung 2 ist vorzugsweise speziell für die Zwecke der Ohrentrocknung konzipiert und kann vorzugsweise tragbar ausgebildet sein.

Fig. 5b zeigt das erfindungsgemäße Ohrentrocknungsgerät umfassend die Vorrichtung 2 zur Generierung eines Luftstromes sowie den erfindungsgemäßen Aufsatz 1 mit Ohrtrichter 18.

Fig. 5c zeigt das erfindungsgemäße Ohrentrocknungsgerät aus Fig. 5a oder 5b. Der Aufsatz 1 ist dabei von einer Schutzkappe 30 verdeckt, welche insbesondere zum Schutz des Aufsatzes 1 während des Transports der Vorrichtung 2 dient.

Fig. 6a ist eine schematische Darstellung des durch den Aufsatz 1 bedingten Strömungsverlaufes einer erfindungsgemäßen Vorrichtung in deren Betriebszustand. Der abgebildete Strömungsverlauf im Inneren des äußeren Gehörgangs 3 entsteht dabei unabhängig von der konkreten Ausführungsform des erfindungsgemäßen Aufsatzes 1 gleichermaßen. Aufgrund der vergleichsweise hohen Einführtiefe des Aufsatzes 1 in den äußeren Gehörgang 3 tritt keine Reflexion des aus dem Aufsatz 1 austretenden Luftstromes an den Seitenwänden 8,9 auf; der Luftstrom wird aufgrund der Leitschaufel 5 beim Verlassen des Aufsatzes 1 verwirbelt, sodass sich im Inneren des äußeren Gehörgangs 3 ein für die Trocknung günstiges Wirbelfeld einstellt.

Fig. 6b zeigt ebenfalls den in den Gehörgang 3 eingeführten Aufsatz 1, allerdings mit einer geringeren Einführtiefe als in Fig. 6a. Dadurch ergibt sich ein anderes Strömungsbild im Inneren des äußeren Gehörgangs 3, welches aber wiederum unabhängig von der gewählten Ausführungsform des Aufsatzes 1 ist. In diesem Fall wird der aus dem Aufsatz 1 austretende Luftstrom an den Seitenwänden 8,9 des äußeren Gehörgangs 3 reflektiert, sodass sich im Inneren des äußeren Gehörgangs 3 ein für die Trocknung günstiges Wirbelfeld einstellt.

Fig. 6c zeigt den in den Gehörgang 3 eingeführten Aufsatz 1, wobei das Ohrentrocknungsgerät jedoch mit anderen Betriebsparametern betrieben wird, was dazu führt, dass der Austrittswinkel des Luftstromes aus dem Aufsatz 1 ein anderer ist. Dadurch ergibt sich ein anderes Strömungsbild im Inneren des äußeren Gehörgangs 3, welches aber wiederum unabhängig von der gewählten Ausführungsform des Aufsatzes 1 ist. Auch in diesem Fall wird der aus dem Aufsatz 1 austretende Luftstrom an den Seitenwänden 8,9 des äußeren Gehörgangs 3 reflektiert, sodass sich im Inneren des äußeren Gehörgangs 3 ein für die Trocknung günstiges Wirbelfeld einstellt.

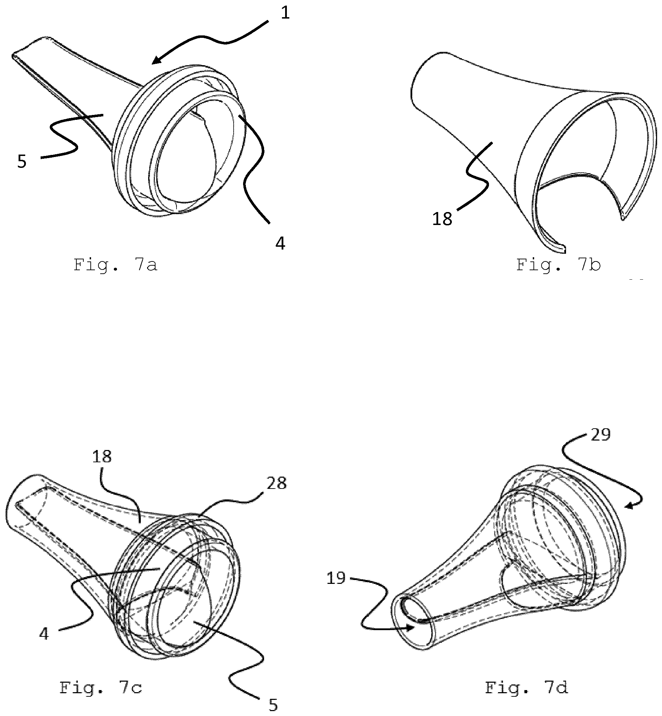

Die Figuren 7a, 7b, 7c und 7c sind jeweils Liniendarstellungen der Figuren 1a, 1b, 2a und 2b.

Fig. 8a, 8b, 8c und 8d zeigen eine vierte Ausführungsform des erfindungsgemäßen Aufsatzes 1. Sämtliche der oben im Zusammenhang mit der ersten, zweiten und dritten Ausführungsform getätigten Aussagen treffen auch auf die hier dargestellte, vierte Ausführungsform zu. Darüber hinaus weist der Verbindungsstutzen 4 des Aufsatzes der vierten Ausführungsform mehrere Vertiefungen 32 auf. Dabei sind die Vertiefungen in dem vorrichtungsseitigen Endabschnitt des Verbindungsstutzens 4 angeordnet, welcher Endabschnitt auch den Rastvorsprung 17 ausbildet. Wie aus Fig. 8c und 8d ersichtlich, weisen die Vertiefungen 32 einen Winkelabstand von 45° auf; dementsprechend sind insgesamt acht Vertiefungen 32 in dem Endabschnitt des Verbindungsstutzens 4 angeordnet.

Wenn der Aufsatz 1 mit dem Endabschnitt des Verbindungsstutzens 4 in die Auslassöffnung 15 der Vorrichtung 2 eingeschoben ist, um den Aufsatz 1 mit der Vorrichtung 2 zu verbinden, verhindert zwar der mit dem Rückhalteabschnitt 34 der Vorrichtung 2 in Eingriff stehende Rastvorsprung 17 des Verbindungsstutzens 4 die unabsichtliche Lösung des Aufsatzes 1 von der Vorrichtung 2; allerdings kann der Aufsatz 1 beliebig gegen die Vorrichtung 2 verdreht werden, sodass - je nach Drehstellung - unterschiedliche Orientierungen der Leitschaufel 5 realisierbar sind. Um den Aufsatz 1 in einer bestimmten Drehposition an der Vorrichtung 2 zu arretieren, ist an der Vorrichtung 2, vorzugsweise im Bereich der Auslassöffnung 15, zumindest eine Rastnase 33 vorgesehen (siehe Fig. 9a und 9b), welche Rastnase 33 mit einer der Vertiefungen 32 in Eingriff gebracht werden können. Die Vertiefungen 32 kennzeichnen somit bevorzugte Drehpositionen des Aufsatzes 1 an der Vorrichtung 2.

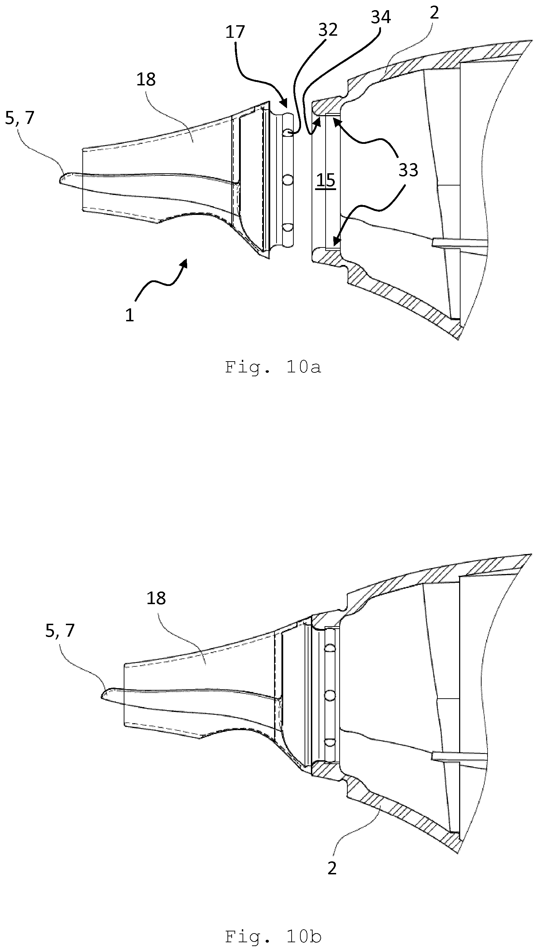

Fig. 10a zeigt den Aufsatz 1 vor der Anbindung an die Vorrichtung 2 zur Abgabe eines Fluids, wie beispielsweise Luft, Wasser oder einer anderen therapeutischen oder pflegenden Flüssigkeit. Um den Aufsatz 1 mit der Vorrichtung 2 zu verbinden, wie dies in Fig. 10b gezeigt ist, kann der Aufsatz 1 mit seinem vorrichtungsseitigen Endabschnitt in die Auslassöffnung 15 eingeschoben werden. Der Rückhalteabschnitt 34 der Vorrichtung 2 verhindert ein unbeabsichtigtes Lösen des Aufsatzes 1 von der Vorrichtung 2. Die Rastnase 33 kann bei bestimmten, zu den Vertiefungen 32 korrespondierenden Drehstellungen des Aufsatzes 1 in die jeweilige Vertiefung 32 eingreifen, um den Aufsatz 1 in der entsprechenden Drehstellung zu fixieren. Durch verdrehen des Aufsatzes 1 aus dieser Drehstellung heraus kann der Aufsatz 1 in eine andere Drehstellung gebracht werden, in welcher die Rastnase 33 in eine andere Vertiefung 32 eingreifen kann.

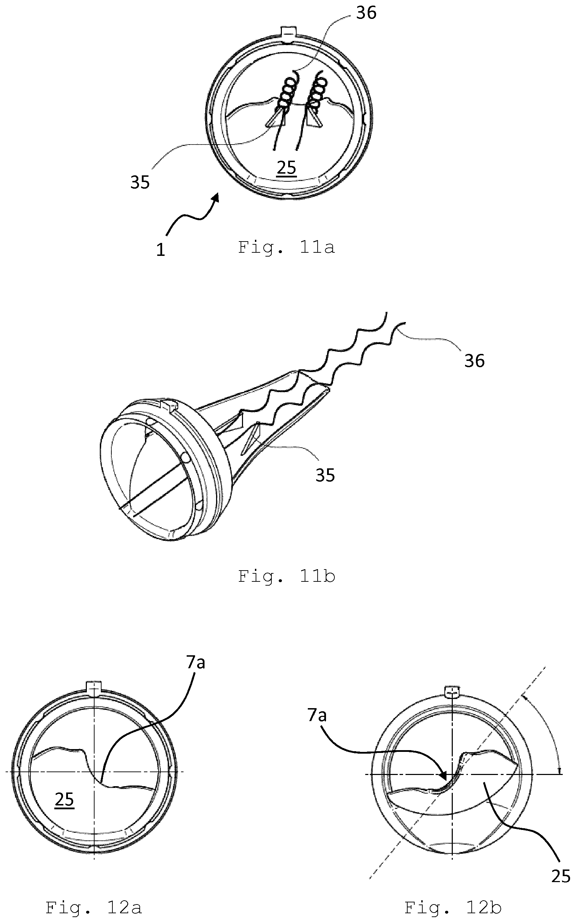

Fig. 11a und 11b zeigen eine fünfte Ausführungsform des erfindungsgemäßen Aufsatzes 1. Sämtliche der oben im Zusammenhang mit der ersten, zweiten, dritten und vierten Ausführungsform getätigten Aussagen treffen auch auf die hier dargestellte, fünfte Ausführungsform zu. Darüber hinaus sind auf dem Leitschaufelboden 25 der Leitschaufel 5 des Aufsatzes 1 zwei Anströmelemente 35 angeordnet.

Die Anströmelemente 35 haben dabei jeweils die Form eines rechtwinkeligen Dreiecks, wie aus Fig. 11a und 11b deutlich hervorgeht, und sind in etwa in einem mittleren Längsabschnitt der Leitschaufel 5 angeordnet. Die Anströmelemente 35 setzen dem durch den Verbindungsstutzen 4 in den Aufsatz 1 einströmenden Fluidstrom Anströmflächen entgegen, die bevorzugter Weise quer zur Strömungsrichtung des anliegenden Fluidstroms verlaufen, sodass die anliegende Strömung des Fluids gestört und in eine Wirbelbewegung gezwungen wird; es bilden sich ein oder mehrere Wirbel 36 (Vortex) des in den Gehörgang eingeleiteten Fluids aus. Wie aus Fig. 11b deutlich zu sehen ist, können die Anströmelemente 35 derart auf dem Leitschaufelboden 25 angeordnet sein, dass die Anströmelemente 35 einander zugewandt sind und sich der Abstand zwischen den Anströmelementen 35 zum proximalen Ende 7 der Leitschaufel 5 hin verringert.

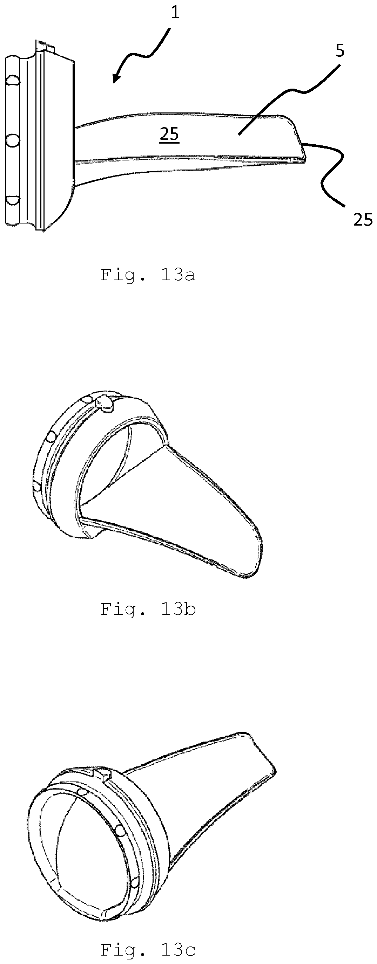

Fig. 12a, 12b, 13a, 13b und 13c zeigen eine sechste Ausführungsform des erfindungsgemäßen Aufsatzes 1. Sämtliche der oben im Zusammenhang mit der ersten, zweiten, dritten, vierten und fünften Ausführungsform getätigten Aussagen treffen auch auf die hier dargestellte, sechste Ausführungsform zu. Darüber hinaus ist der Leitschaufelboden 25 dieser Ausführungsform nicht symmetrisch, wie dies bei den anderen der oben beschriebenen Ausführungsformen der Fall ist, sondern asymmetrisch ausgebildet.

Dies kann dadurch erreicht werden, dass einander gegenüberliegende Abschnitte der Leitschaufelwangen 26, 27 jeweils eine unterschiedliche Höhe aufweisen, wie dies erfindungsgemäß vorgesehen ist, oder dass der Leitschaufelboden 25 im Bereich eines zweiten Längsabschnitts des Leitschaufelbodens 25 gegenüber einem ersten Längsabschnitt verschoben und/oder verdreht ist. Insbesondere kann der in die zweite Richtung gekrümmte zweite Abschnitt 5b der Leitschaufel 25 wie gezeigt gegenüber dem in die erste Richtung gekrümmten ersten Abschnitt 5a der Leitschaufel 25 verdreht sein.

### BEZUGSZEICHENLISTE

- 1: Aufsatz
- 2: Vorrichtung
- 3: äußerer Gehörgang
- 4: Verbindungsstutzen
- 5: zungenartig ausgebildete Leitschaufel
- 5a: erster Abschnitt der Leitschaufel
- 5b: zweiter Abschnitt der Leitschaufel
- 5c: im Verbindungsstutzen verlaufender Abschnitt der Leitschaufel
- 6: distales Ende der Leitschaufel
- 7: proximales Ende der Leitschaufel
- 7a: proximales Ende des Leitschaufelbodens
- 8: obere Seitenwand des Gehörgangs
- 9: untere Seitenwand des Gehörgangs
- 10: Leitkanal
- 11: Recessus meatus acustici externi
- 12: Längsachse
- 13: erster Scheitelpunkt
- 14: zweiter Scheitelpunkt
- 15: Auslassöffnung
- 16: längsseitiger Rand
- 17: Rastvorsprung
- 18: Ohrtrichter
- 19: proximale Öffnung
- 20: Zuluftkanal
- 21: Abluftkanal
- 22: Ausströmöffnung
- 23: Einströmöffnung
- 24: Auslass
- 25: Leitschaufelboden
- 26: erste Leitschaufelwange
- 27: zweite Leitschaufelwange
- 28: Abstandhalter
- 29: distale Öffnung des Verbindungsstutzens
- 30: Schutzkappe
- 31: Trommelfell
- 32: Vertiefung
- 33: Rastnase
- 34: Rückhalteabschnitt
- 35: Anströmelement
- 36: Wirbel

## Patentansprüche

1. Aufsatz (1) für eine Vorrichtung (2) zur Generierung eines Luftstromes, vorzugsweise eines Warmluftstromes, oder zur Abgabe eines Fluids, welcher Aufsatz (1) dazu eingerichtet ist, zumindest abschnittsweise in einen äußeren Gehörgang (3) eines menschlichen oder tierischen Ohres eingeführt zu werden, wobei der Aufsatz (1)
- einen Verbindungsstutzen (4) zur Anbindung an die Vorrichtung (2) aufweist, wobei der Aufsatz (1)
- eine von dem Verbindungsstutzen (4) abstehende, zungenartig ausgebildete Leitschaufel (5) zur Führung von aus der Vorrichtung (2) ausströmender Luft oder von der Vorrichtung abgegebenem Fluid, mit einem Leitschaufelboden (25)
aufweist, und wobei die Leitschaufel (5) dem Verbindungsstutzen (4) in Strömungsrichtung des Luftstromes oder Fluids nachgeordnet ist, wobei die zungenartig ausgebildete Leitschaufel (5) zwei von dem Leitschaufelboden (25) abstehende Leitschaufelwangen, nämlich eine erste Leitschaufelwange (26) und eine zweite Leitschaufelwange (27), aufweist, wobei Leitschaufelboden (25) und Leitschaufelwangen (26,27) einen Leitkanal oder einen Teil eines Leitkanals für die aus der Vorrichtung (2) zur Generierung eines Luftstroms ausströmende Luft ausbilden, **dadurch gekennzeichnet, dass** einander gegenüberliegende Abschnitte der Leitschaufelwangen (26,27) jeweils eine unterschiedliche Höhe aufweisen.

2. Aufsatz (1) nach Anspruch 1, **dadurch gekennzeichnet, dass** der Aufsatz (1) einen Ohrtrichter (18) umfasst, welcher Ohrtrichter (18) die zungenartig ausgebildete Leitschaufel (5) zumindest abschnittsweise, vorzugsweise gänzlich, der Länge nach umhüllt und eine proximale Öffnung (19) aufweist, mit welcher proximalen Öffnung (19) der Aufsatz (1) bei bestimmungsgemäßer Verwendung in den äußeren Gehörgang (3) einführbar oder ansetzbar ist, sodass die proximale Öffnung (19) die dem Trommelfell (31) nächstgelegene Öffnung des Ohrtrichters (18) darstellt, wobei der Ohrtrichter (18) an den Verbindungsstutzen (4) anschließt und von diesem absteht, oder der Verbindungsstutzen (4) einen Endabschnitt des Ohrtrichters (18) ausbildet.

3. Aufsatz (1) nach einem der Ansprüche 1 oder 2, **dadurch gekennzeichnet, dass** der Leitschaufelboden (25) eines ersten Abschnitts (5a) der Leitschaufel (5) einen Längsquerschnittsverlauf aufweist, der in eine erste Richtung gekrümmt ist, und der Leitschaufelboden (25) eines zweiten Abschnitts (5b) der Leitschaufel (5) einen Längsquerschnittsverlauf aufweist, der in eine zweite Richtung gekrümmt ist, und die beiden gekrümmten Abschnitte (5a,5b) zu einander entgegengesetzt gekrümmt verlaufen.

4. Aufsatz (1) nach einem der Ansprüche 2 oder 3, **dadurch gekennzeichnet, dass**
- der Verbindungsstutzen (4) und die Leitschaufel (5) einstückig ausgebildet sind, und vorzugsweise als Einheit mit dem Ohrtrichter (18) verbindbar sind, oder dass
- der Ohrtrichter (18) und die Leitschaufel (5) einstückig ausgebildet sind, und vorzugsweise als Einheit mit dem Verbindungsstutzen (4) verbindbar sind, oder dass
- der Verbindungsstutzen (4), die Leitschaufel (5) und der Ohrtrichter (18) einstückig ausgebildet sind.

5. Aufsatz (1) nach einem der Ansprüche 1 bis 4, **dadurch gekennzeichnet, dass** der Verbindungsstutzen (4) dazu eingerichtet ist, den Aufsatz (1) verdrehbar an die Vorrichtung (2) anzubinden, wobei der Verbindungsstutzen (4) an einer äußeren Mantelfläche zumindest eine Vertiefung (32) aufweist, welche Vertiefung (32) mit zumindest einer Rastnase (33) der Vorrichtung (2) in Eingriff bringbar ist, um den Aufsatz (1) in einer bestimmten Drehposition an der Vorrichtung (2) zu arretieren.

6. Aufsatz (1) nach Anspruch 5, **dadurch gekennzeichnet, dass** mehrere Vertiefungen (32) voneinander beabstandet auf der äußeren Mantelfläche des Verbindungsstutzens (4) angeordnet sind, um den Aufsatz (1) in verschiedenen Drehpositionen an der Vorrichtung (2) arretieren zu können.

7. Aufsatz (1) nach Anspruch 6, **dadurch gekennzeichnet, dass** die Vertiefungen (32) einen Winkelabstand von 180°, vorzugsweise von 90°, besonders bevorzugt von 45°, zueinander aufweisen.

8. Aufsatz (1) nach einem der Ansprüche 1 bis 7, **dadurch gekennzeichnet, dass** zumindest ein Anströmelement (35) vorgesehen ist, welches Anströmelement (35) von dem Leitschaufelboden (25) abragt, um das aus der Vorrichtung (2) ausströmende Fluid zu verwirbeln.

9. Aufsatz (1) nach Anspruch 8, **dadurch gekennzeichnet, dass** zumindest zwei, vorzugsweise flächig ausgebildete, Anströmelemente (35) vorgesehen sind, welche einander gegenüberliegend auf dem Leitschaufelboden (25) angeordnet sind.

10. Aufsatz (1) nach einem der Ansprüche 8 oder 9, **dadurch gekennzeichnet, dass** die Anströmelemente (35) die Form eines, vorzugsweise rechtwinkeligen, Dreiecks aufweisen.

11. Aufsatz (1) nach einem der Ansprüche 9 oder 10, **dadurch gekennzeichnet, dass** Anströmflächen der Anströmelemente (35), welche Anströmflächen dem Verbindungsstutzen zugewandt sind, schräg zueinander verlaufen.

12. Aufsatz (1) nach einem der Ansprüche 9 bis 11, **dadurch gekennzeichnet, dass,** von dem Verbindungsstutzen (4) aus betrachtet, ein Abstand zwischen gegenüberliegenden Anströmelementen (35) in Längsrichtung des Aufsatzes (1) abnimmt.

13. Aufsatz (1) nach einem der Ansprüche 1 bis 12, **dadurch gekennzeichnet, dass** der Leitschaufelboden (25) zumindest im Bereich eines zweiten Längsabschnitts der Leitschaufel (5), vorzugsweise seines zweiten Abschnitts (5b), gegenüber einem ersten Längsabschnitt der Leitschaufel (5), vorzugsweise dem ersten Abschnitt (5a), verdreht ist.

14. Ohrentrocknungsgerät umfassend eine Vorrichtung (2) zur Generierung eines Luftstromes sowie einen Aufsatz (1) nach einem der vorhergehenden Ansprüche.

15. Gerät zur pflegenden oder therapeutischen Behandlung des äußeren Gehörgangs eines menschlichen oder tierischen Ohres umfassend eine Vorrichtung zur Abgabe eines Fluids sowie einen Aufsatz (1) nach einem der vorhergehenden Ansprüche.

## Claims

1. Attachment (1) for a device (2) for generating an airflow, preferably a warm airflow, or for dispensing a fluid, which attachment (1) is adapted to be introduced at least in sections into an external ear canal (3) of a human or animal ear, wherein the attachment (1) comprises
- a connecting piece (4) for connection to the device (2), wherein the attachment (1) comprises
- a tongue-like guide vane (5), projecting from the connecting piece (4), for guiding air flowing out of the device (2) or fluid emitted by the device, having a guide vane base (25),
and wherein the guide vane (5) is arranged downstream of the connecting piece (4) in the direction of flow of the airflow or fluid, wherein the tongue-like guide vane (5) has two guide vane cheeks projecting from the guide vane base (25), namely a first guide vane cheek (26) and a second guide vane cheek (27), wherein guide vane base (25) and guide vane cheeks (26, 27) form a guide channel or part of a guide channel for the air flowing out of the device (2) for generating an airflow, **characterized in that** opposing sections of the guide vane cheeks (26, 27) each have a different height.

2. Attachment (1) according to claim 1, **characterized in that** the attachment (1) comprises an ear funnel (18), which ear funnel (18) envelops the tongue-like guide vane (5) at least in sections, preferably completely, along its length and has a proximal opening (19) with which proximal opening (19) the attachment (1) is insertable into or attachable to the external ear canal (3) when used as intended, such that the proximal opening (19) represents the opening of the ear funnel (18) closest to the eardrum (31), wherein the ear funnel (18) adjoins the connecting piece (4) and projects therefrom, or the connecting piece (4) forms an end section of the ear funnel (18).

3. Attachment (1) according to one of claims 1 or 2, **characterized in that** the guide vane base (25) of a first section (5a) of the guide vane (5) has a longitudinal cross-sectional profile curved in a first direction, and the guide vane base (25) of a second section (5b) of the guide vane (5) has a longitudinal cross-sectional profile curved in a second direction, and the two curved sections (5a, 5b) extend in an adversely curved manner to one another.

4. Attachment (1) according to one of claims 2 or 3, **characterized in that**
- the connecting piece (4) and the guide vane (5) are formed in one piece and can preferably be connected as a unit to the ear funnel (18), or that
- the ear funnel (18) and the guide vane (5) are formed in one piece and can preferably be connected as a unit to the connecting piece (4), or that
- the connecting piece (4), the guide vane (5) and the ear funnel (18) are formed in one piece.

5. Attachment (1) according to one of claims 1 to 4, **characterized in that** the connecting piece (4) is adapted to rotatably connect the attachment (1) to the device (2), wherein the connecting piece (4) has at least one recess (32) on an outer circumferential surface, which recess (32) can be brought into engagement with at least one latching lug (33) of the device (2) in order to lock the attachment (1) in a specific rotational position on the device (2).

6. Attachment (1) according to claim 5, **characterized in that** a plurality of recesses (32) are arranged at a distance from one another on the outer lateral surface of the connecting piece (4) in order to be able to lock the attachment (1) in various rotational positions on the device (2).

7. Attachment (1) according to claim 6, **characterized in that** the recesses (32) have an angular spacing of 180°, preferably of 90°, particularly preferably of 45°, from one another.

8. Attachment (1) according to one of claims 1 to 7, **characterized in that** at least one inflow element (35) is provided, which inflow element (35) projects from the guide vane base (25) in order to swirl the fluid flowing out of the device (2).

9. Attachment (1) according to claim 8, **characterized in that** at least two, preferably flat, inflow elements (35) are provided, which are arranged opposite one another on the guide vane base (25).

10. Attachment (1) according to one of claims 8 or 9, **characterized in that** the inflow elements (35) have the shape of a, preferably right-angled, triangle.

11. Attachment (1) according to one of claims 9 or 10, **characterized in that** inflow surfaces of the inflow elements (35), which inflow surfaces face the connecting piece, extend obliquely to one another.

12. Attachment (1) according to one of claims 9 to 11, **characterized in that,** as viewed from the connecting piece (4), a distance between opposing inflow elements (35) decreases in the longitudinal direction of the attachment (1).

13. Attachment (1) according to one of claims 1 to 12, **characterized in that** the guide vane base (25) is twisted at least in the region of a second longitudinal section of the guide vane (5), preferably its second section (5b), relative to a first longitudinal section of the guide vane (5), preferably the first section (5a).

14. Ear drying apparatus, comprising a device (2) for generating an airflow and an attachment (1) according to one of the preceding claims.

15. Apparatus for the caring or therapeutic treatment of the external auditory canal of a human or animal ear comprising a device for dispensing a fluid and an attachment (1) according to one of the preceding claims.

## Revendications

1. Embout (1) pour un dispositif (2) destiné à produire un flux d'air, de préférence un flux d'air chaud, ou à émettre un fluide, lequel embout (1) est conçu pour être introduit au moins en partie dans le conduit auditif externe (3) d'une oreille humaine ou animale, l'embout (1) comportant
- un raccord d'assemblage (4) pour la liaison au dispositif (2), l'embout (1) comportant
- une pale directrice (5) en forme de languette dépassant du raccord d'assemblage (4) pour guider de l'air sortant du dispositif (2) ou du fluide sortant du dispositif, avec un fond de pale directrice (25),
et la pale directrice (5) se trouvant en aval du raccord d'assemblage (4) dans le sens d'écoulement du flux d'air ou du fluide, dans lequel la pale directrice (5) en forme de languette présente deux joues de pale directrice qui dépassent du fond de pale directrice (25), à savoir une première joue de pale directrice (26) et une deuxième joue de pale directrice (27), le fond de pale directrice (25) et les joues de pale directrice (26, 27) formant un conduit ou une partie de conduit pour l'air sortant du dispositif (2) afin de produire un flux d'air, **caractérisé en ce que** des parties qui se font face des joues de pale directrice (26, 27) ont une hauteur différente.

2. Embout (1) selon la revendication 1, **caractérisé en ce que** l'embout (1) comprend un entonnoir auriculaire (18), lequel entonnoir auriculaire (18) entoure la pale directrice (5) en forme de languette au moins en partie, de préférence sur toute sa longueur, et présente une ouverture proximale (19), laquelle ouverture proximale (19) permet d'introduire ou de poser l'embout (1) dans le conduit auditif externe (3) lorsqu'il est utilisé correctement, de sorte que l'ouverture proximale (19) représente l'ouverture de l'entonnoir auriculaire (18) la plus proche du tympan (31), l'entonnoir auriculaire (18) se raccordant au raccord d'assemblage (4) et dépassant de celui-ci ou le raccord d'assemblage (4) formant une partie d'extrémité de l'entonnoir auriculaire (18).

3. Embout (1) selon l'une des revendications 1 ou 2, **caractérisé en ce que** le fond de pale directrice (25) d'une première partie (5a) de la pale directrice (5) a une forme en coupe longitudinale incurvée dans une première direction et le fond de pale directrice (25) d'une deuxième partie (5b) de la pale directrice (5) a une forme en coupe longitudinale incurvée dans une deuxième direction, et les deux parties incurvées (5a, 5b) sont incurvées en sens inverse l'une de l'autre.

4. Embout (1) selon l'une des revendications 2 ou 3, **caractérisé en ce que**
- le raccord d'assemblage (4) et la pale directrice (5) sont formés d'une pièce et peuvent de préférence être assemblés comme une unité à l'entonnoir auriculaire (18) ou **en ce que**
- l'entonnoir auriculaire (18) et la pale directrice (5) sont formés d'une pièce et peuvent de préférence être assemblés comme une unité au raccord d'assemblage (4) ou **en ce que**
- le raccord d'assemblage (4), la pale directrice (5) et l'entonnoir auriculaire (18) sont formés d'une pièce.

5. Embout (1) selon l'une des revendications 1 à 4, **caractérisé en ce que** le raccord d'assemblage (4) est conformé de façon à relier l'embout (1) au dispositif (2) avec possibilité de rotation, le raccord d'assemblage (4) présentant au moins un creux (32) sur une surface d'enveloppe extérieure, lequel creux (32) peut être mis en prise avec au moins un ergot d'enclenchement (33) du dispositif (2) afin de retenir l'embout (1) dans une certaine position de rotation sur le dispositif (2).

6. Embout (1) selon la revendication 5, **caractérisé en ce que** plusieurs creux (32) sont disposés à distance les uns des autres sur la surface d'enveloppe extérieure du raccord d'assemblage (4) afin de retenir l'embout (1) dans différentes positions de rotation sur le dispositif (2).

7. Embout (1) selon la revendication 6, **caractérisé en ce que** les creux (32) sont écartés les uns des autres d'une distance angulaire de 180°, de préférence de 90°, en particulier de 45°.

8. Embout (1) selon l'une des revendications 1 à 7, **caractérisé en ce qu'**au moins un élément de chicane (35) est prévu, lequel élément de chicane (35) dépasse du fond de pale directrice (25) afin de créer des turbulences dans le fluide sortant du dispositif (2).

9. Embout (1) selon la revendication 8, **caractérisé en ce qu'**au moins deux éléments de chicane (35), de préférence plats, sont prévus et disposés l'un en face de l'autre sur le fond de pale directrice (25).

10. Embout (1) selon l'une des revendications 8 ou 9, **caractérisé en ce que** les éléments de chicane (35) sont en forme de triangle, de préférence de triangle rectangle.

11. Embout (1) selon l'une des revendications 9 ou 10, **caractérisé en ce que** les surfaces d'attaque des éléments de chicane (35) qui sont tournées vers le raccord d'assemblage sont obliques l'une vers l'autre.

12. Embout (1) selon l'une des revendications 9 à 11, **caractérisé en ce que** la distance entre les éléments de chicane (35) qui se font face diminue, vue à partir du raccord d'assemblage (4), dans le sens longitudinal de l'embout (1).

13. Embout (1) selon l'une des revendications 1 à 12, **caractérisé en ce que** le fond de pale directrice (25) est vrillé au moins dans un deuxième segment de longueur de la pale directrice (5), de préférence dans sa deuxième partie (5b), par rapport à un premier segment de longueur de la pale directrice (5), de préférence la première partie (5a).

14. Appareil de séchage auriculaire comprenant un dispositif (2) pour la production d'un flux d'air ainsi qu'un embout (1) selon l'une des revendications précédentes.

15. Appareil pour le soin ou le traitement du conduit auditif externe d'une oreille humaine ou animale, comprenant un dispositif émettant un fluide ainsi qu'un embout (1) selon l'une des revendications précédentes.
